(19) **Europäisches Patentamt / European Patent Office / Office européen des brevets**

(11) **EP 4 018 918 B1**

(12) **EUROPEAN PATENT SPECIFICATION**

(45) Date of publication and mention
of the grant of the patent:
**26.03.2025 Bulletin 2025/13**

(21) Application number: **22156356.2**

(22) Date of filing: **27.02.2015**

(51) International Patent Classification (IPC):
**A61B 5/00** (2006.01)

(52) Cooperative Patent Classification (CPC):
**A61B 5/0095;** A61B 5/742; A61B 2562/0204;
A61B 2576/00; G16H 30/40

(54) **PROBE HAVING LIGHT DELIVERY THROUGH COMBINED OPTICALLY DIFFUSING AND ACOUSTICALLY PROPAGATING ELEMENT**

SONDE MIT LICHTABGABE DURCH KOMBINIERTE OPTISCH STREUENDE UND AKUSTISCH AUSBREITENDE ELEMENTE

SONDE PRÉSENTANT UNE DISTRIBUTION DE LUMIÈRE À TRAVERS UN ÉLÉMENT COMBINÉ DIFFUSEUR OPTIQUEMENT ET PROPAGATEUR ACOUSTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2014 US 201461945650 P**

(43) Date of publication of application:
**29.06.2022 Bulletin 2022/26**

(62) Document number(s) of the earlier application(s) in accordance with Art. 76 EPC:
**15754967.6 / 3 110 312**

(73) Proprietor: **Seno Medical Instruments, Inc.**
**San Antonio, TX 78230 (US)**

(72) Inventors:
- **ZALEV, Jason**
  **Thornhill (CA)**
- **HERZOG, Donald**
  **Collingswood, NJ (US)**

(74) Representative: **Leach, Sean Adam et al**
**Mathys & Squire**
**The Shard**
**32 London Bridge Street**
**London SE1 9SG (GB)**

(56) References cited:
| | |
|---|---|
| **EP-A1- 2 148 183** | **JP-A- 2013 202 050** |
| **US-A1- 2008 071 172** | **US-A1- 2013 064 771** |
| **US-A1- 2013 109 950** | **US-A1- 2013 197 345** |
| **US-A1- 2013 304 405** | |

- **NIEDERHAUSER J J ET AL: "COMBINED ULTRASOUND AND OPTOACOUSTIC SYSTEM FOR REAL-TIME HIGH-CONTRAST VASCULAR IMAGING IN VIVO", IEEE TRANSACTIONS ON MEDICAL IMAGING, IEEE, USA, vol. 24, no. 4, 1 April 2005 (2005-04-01), pages 436 - 440, XP001240177, ISSN: 0278-0062, DOI: 10.1109/ TMI.2004.843199**

## EP 4 018 918 B1

**Description**

This application is a non-provisional of and claims priority to U.S. Provisional Patent Application No. 61/945,650 filed February 27, 214.

This application includes material which is subject to copyright protection. The copyright owner has no objection to the facsimile reproduction by anyone of the patent disclosure, as it appears in the Patent and Trademark Office files or records, but otherwise reserves all copyright rights whatsoever.

**Field**

The present invention relates in general to the field of medical imaging, and in particular to an optoacoustic probe that provides light delivery through a combined optically diffusing and acoustically propagating element.

Relevant prior art is disclosed in US 2013/197345A1.

The present invention is defined in the appended claims.

According to the present invention there is provided an opto-acoustic probe comprising:an acoustically transmissive optical distribution element comprising:

a distal surface configured to couple to a volume of a biological tissue to deliver optical energy to the volume and to exchange acoustic energy with the volume; and,

a proximal surface proximate to an acoustic receiver,

the acoustically transmissive optical distribution element configured to permit acoustic energy originating within the volume based on the optical energy delivered, to pass through the acoustically transmissive optical distribution element to be detected by the acoustic receiver;

wherein the acoustically transmissive optical distribution element is configured to receive the optical

energy at one or more optical energy inputs from an optical energy path of the probe, and the acoustically transmissive optical distribution element is configured to distribute the optical energy received at the one or more optical energy inputs from the optical energy path to the distal surface, so that the optical energy distributed exits the distal surface of the acoustically transmissive optical distribution element;

wherein the optical distribution element is configured to absorb at least a portion of the optical energy to produce a secondary acoustic return that interferes with a direct acoustic return component originating from the volume, wherein the opto-acoustic probe is operatively connected to an opto-acoustic system comprising a processing unit adapted to separate the secondary acoustic return from the direct acoustic return component using a component separation, and the system comprises a display to display an image based on the separated direct acoustic return component; and, wherein the optical distribution element comprises an optically diffusing fiber.

**Brief Description of the Drawings**

Objects, features, and advantages of the invention will be apparent from the following more particular description of preferred embodiments as illustrated in the accompanying drawings, in which reference characters refer to the same parts throughout the various views. The drawings are not necessarily to scale, emphasis instead being placed upon illustrating principles of the invention.

Figures 1 a) to c) illustrate various shapes that can be used for a combined optical and acoustic port in accordance with an embodiment of the invention.

Figure 2 a) is illustrative of an acoustically transmissive region adjacent to an acoustically non-transmissive region that is optically transmissive.

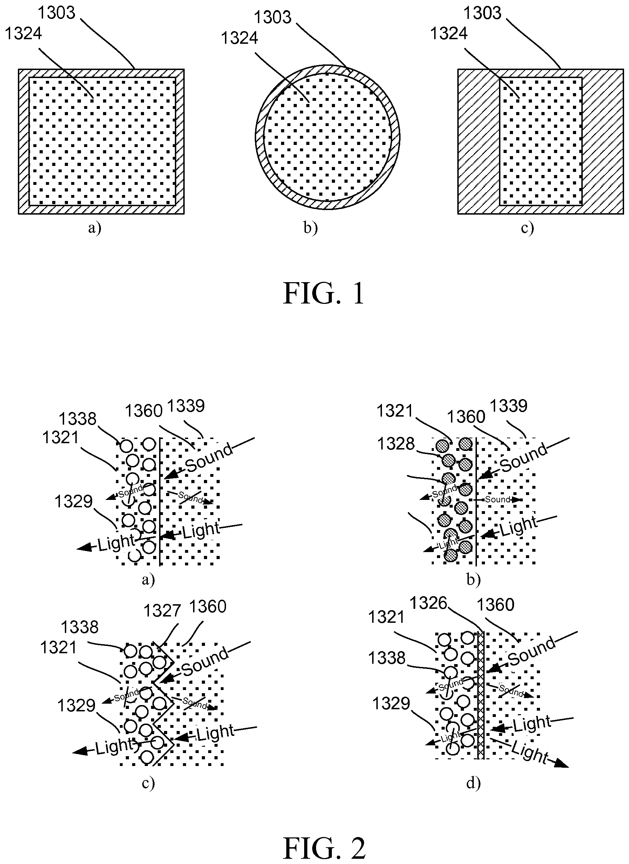

Figure 2 b) is illustrative of an acoustically transmissive region adjacent to an acoustically non-transmissive region that is optically non-transmissive.

Figure 2 c) is illustrative of an acoustically transmissive optical distribution element adjacent to an acoustically non-transmissive region that is optically transmissive.

Figure 2 d) is illustrative of an acoustically transmissive optical distribution element adjacent to an acoustically non-transmissive region.

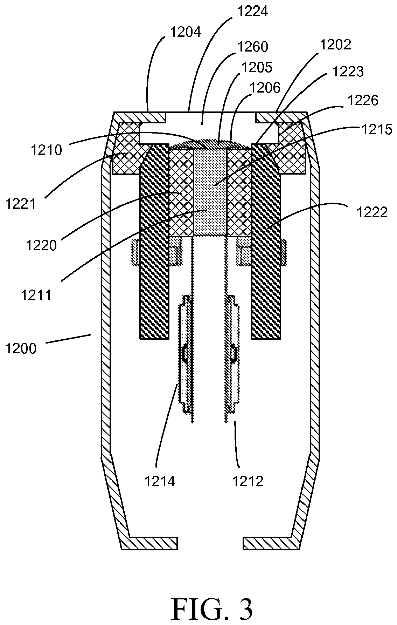

Figure 3 is an illustrative embodiment of an opto-acoustic probe with an acoustically transmissive optical distribution element.

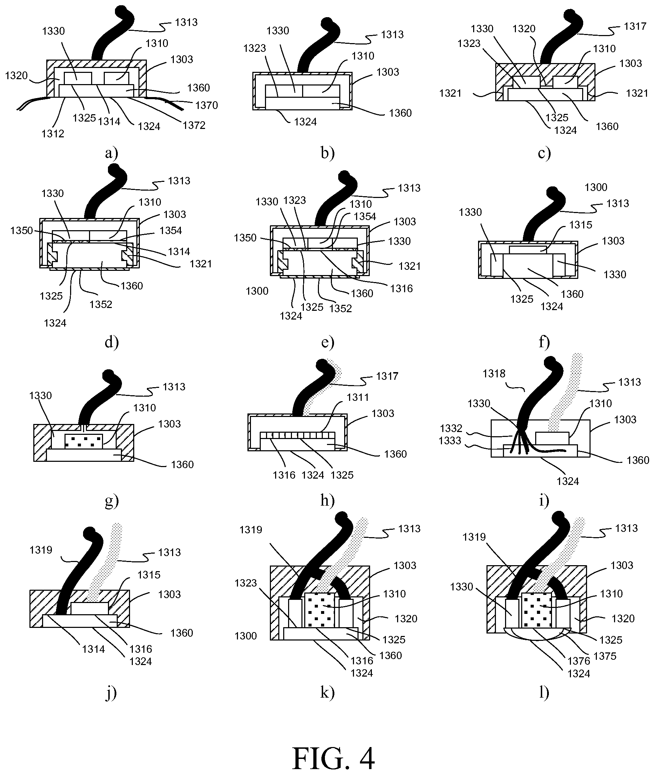

Figures 4 a) to l) are illustrative of numerous embodiments for an opto-acoustic probe with an acoustically transmissive optical distribution element.

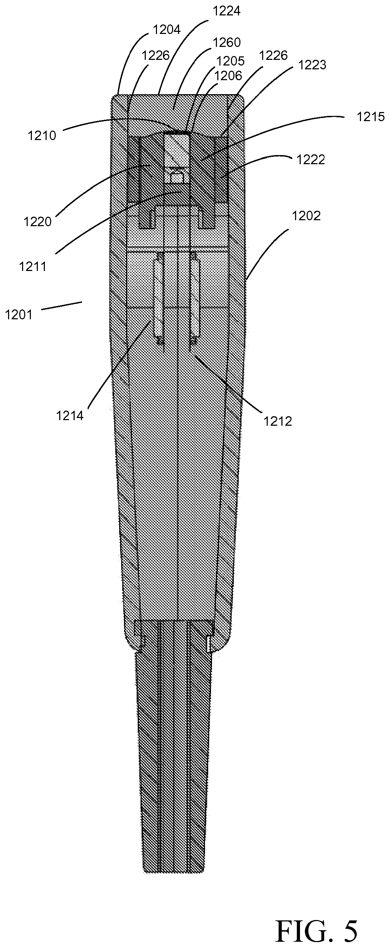

Figure 5 shows an embodiment of an opto-acoustic probe with and acoustically transmissive optical distribution element having an ergonomic form of a conventional ultrasound transducer.

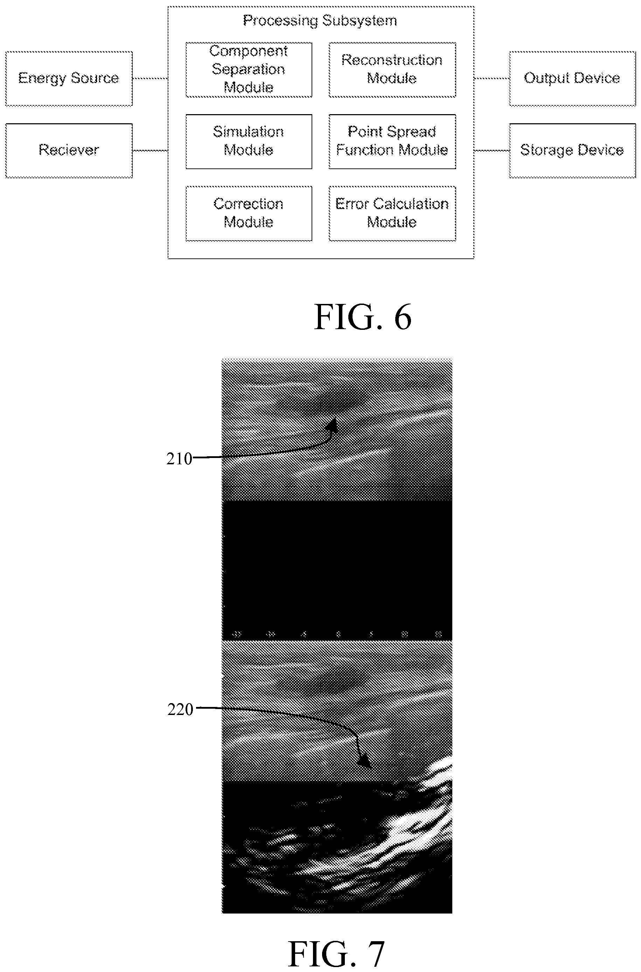

Figure 6 shows a block diagram of an embodiment of a Component Separation System.

Figure 7 shows two images reconstructed from an acoustic signal received from a given volume.

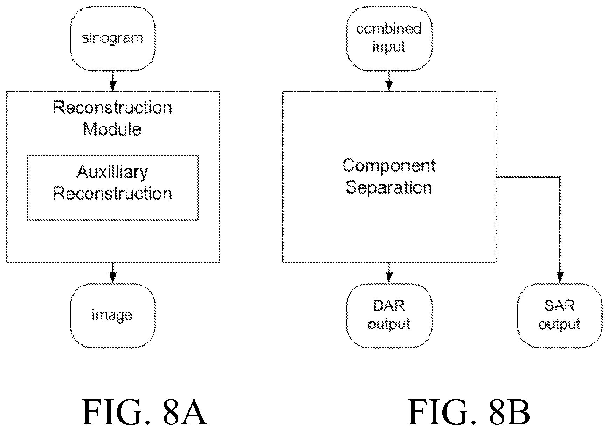

Figure 8A is a block-level process flow chart illustrating the process flow associated with a reconstruction module.

Figure 8B is a block-level process flow chart illustrating an overall component separation process in accordance with an embodiment.

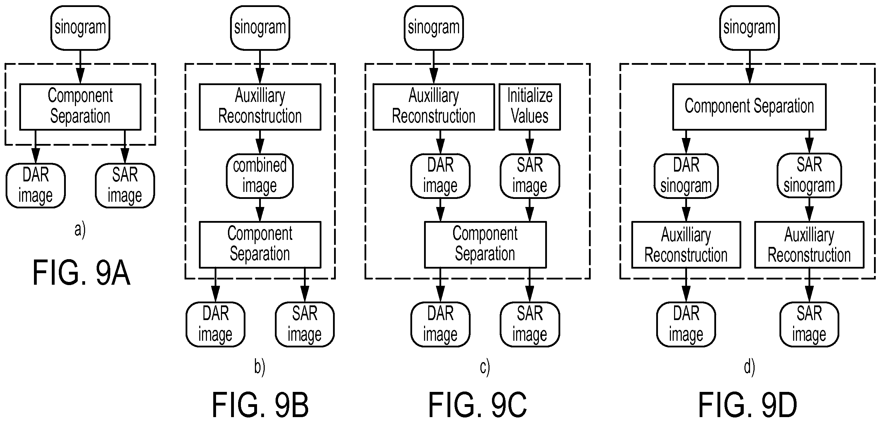

Figures 9A through 9D show examples of applications of reconstruction with component separation.

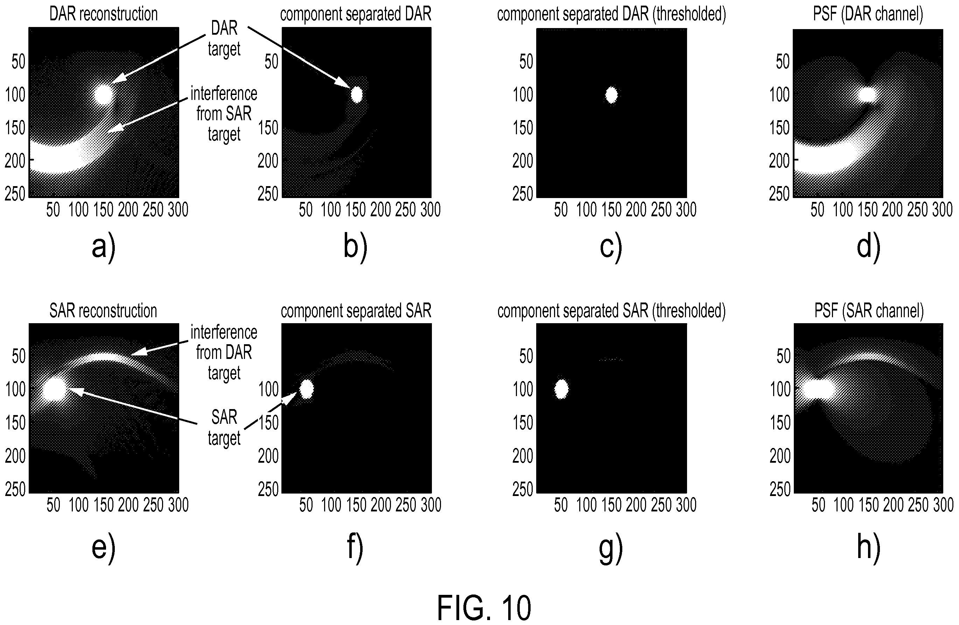

Figure 10 is a series of images showing an example of SAR/DAR component separation applied to a digital phantom with a DAR and SAR target.

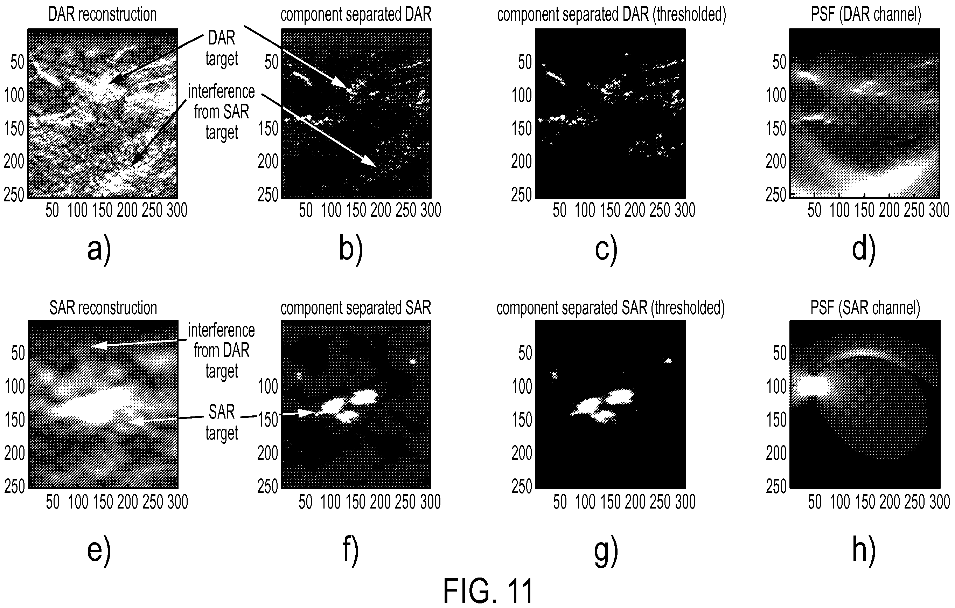

Figure 11 is a series of images showing an example of SAR/DAR component separation applied to data from a breast lesion.

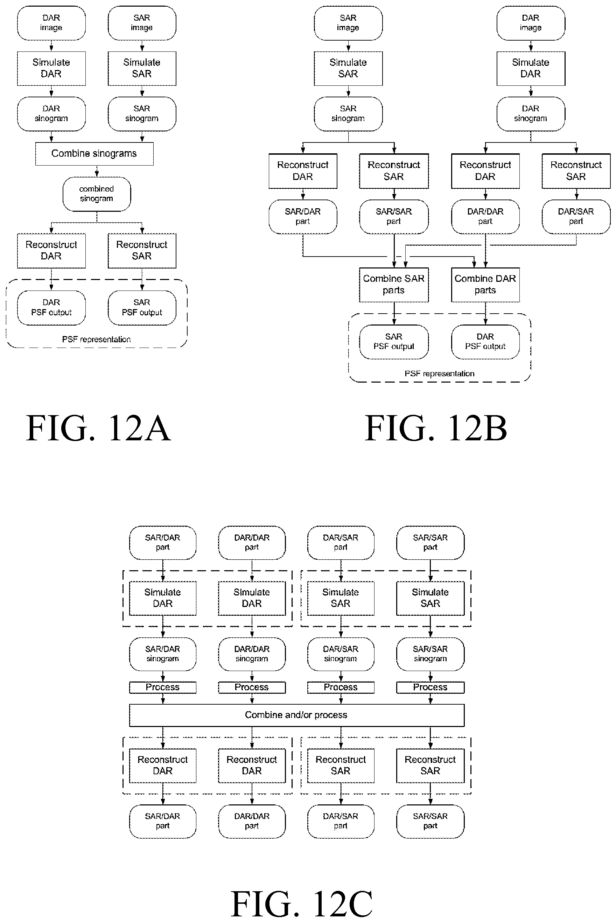

Figures 12a through 12c are block-level process flow charts for three alternative embodiments of aspects of a Point Spread Function (PSF) module.

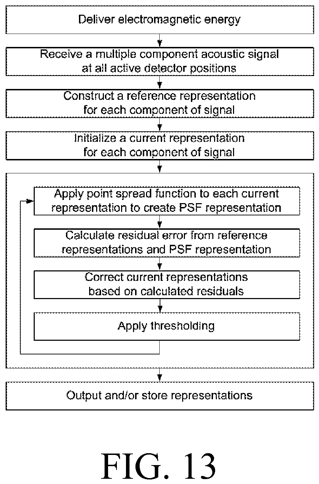

Figure 13 is a flow diagram illustrating a process flow for SAR/DAR component separation in accordance with an embodiment.

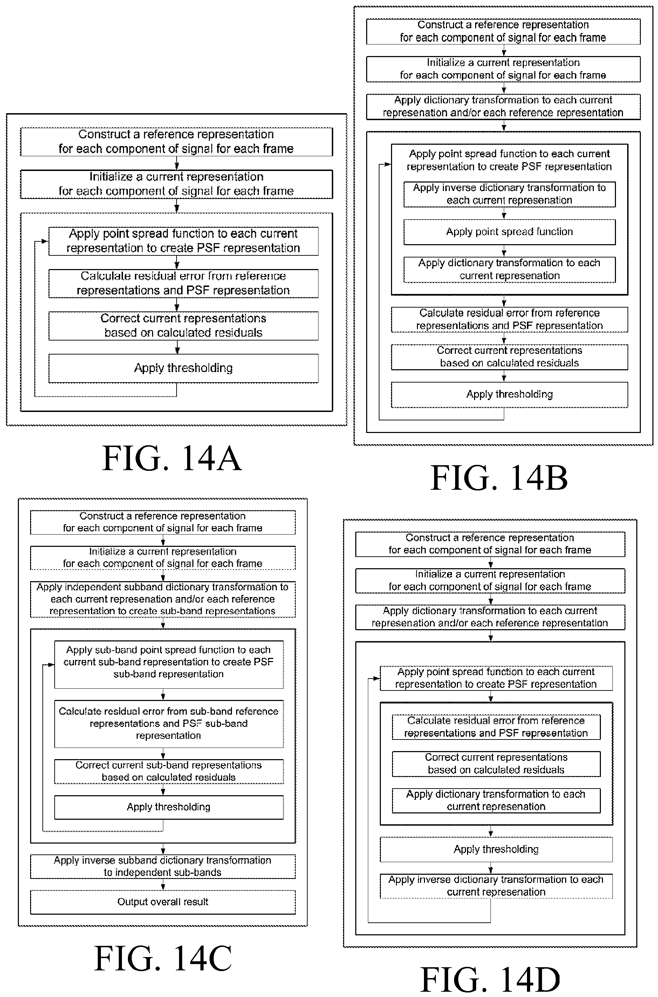

Figures 14A through 14D are block-level flow diagrams showing illustrative embodiments for using sparse representations in component separation.

**Detailed Description**

While the invention is amenable to various modifications and alternative forms, specifics thereof have been shown by way of example in the drawings and will be described in detail.

The following description and drawings are illustrative and are not to be construed as limiting. Numerous specific details are described to provide a thorough understanding. However, in certain instances, well-known or conventional details are not described in order to avoid obscuring the description. References to one or an embodiment in the present disclosure are not necessarily references to the same embodiment; and, such references mean at least one.

Reference in this specification to "one embodiment" or "an embodiment" means that a particular feature, structure, or characteristic described in connection with the embodiment is included in at least one embodiment of the disclosure. The appearances of the phrase "in one embodiment" in various places in the specification are not necessarily all referring to the same embodiment, nor are separate or alternative embodiments mutually exclusive of other embodiments. Moreover, various features are described which may be exhibited by some embodiments and not by others. Similarly, various requirements are described which may be requirements for some embodiments but not other embodiments.

The systems and methods are described below with reference to, among other things, block diagrams, operational illustrations and algorithms of methods and devices to process optoacoustic imaging data. It is understood that each block of the block diagrams, operational illustrations and algorithms and combinations of blocks in the block diagrams, operational illustrations and algorithms, can be implemented by means of analog or digital hardware and computer program instructions.

These computer program instructions can be provided to a processor of a general purpose computer, special purpose computer, ASIC, or other programmable data processing apparatus, such that the instructions, which execute via the processor of the computer or other programmable data processing apparatus, implements the functions/acts specified in the block diagrams, operational block or blocks and or algorithms.

**In** some alternate implementations, the functions/acts noted in the blocks can occur out of the order noted in the operational illustrations. For example, two blocks shown in succession can in fact be executed substantially concurrently or the blocks can sometimes be executed in the reverse order, depending upon the functionality/acts involved.

Reference will now be made in more detail to various embodiments of the present invention, examples of which are illustrated in the accompanying drawings.

Figures 1 a) to c) illustrate various shapes that can be used for a combined optical and acoustic port 1324 in accordance with an embodiment. In this embodiment, light exits the combined optical and acoustic port 1324 with a homogenously constant optical energy over its entire surface area.

Figure 2 a) is illustrative of an acoustically transmissive region 1339, adjacent to an acoustically non-transmissive region 1329 that is optically transmissive. Acoustic waves are dampened in the acoustically non-transmissive region 1329, and optical energy is transmitted through the region 1329. In an embodiment, the acoustically transmissive region 1339 is an acoustically transmissive optical distribution element 1360. In an embodiment, the acoustically non-transmissive region 1329 comprises an acoustically absorbing agent 1338. In an embodiement, the acoustically absorbing agent is microbubbles. In an embodiment, the acoustically absorbing region is an isolator 1321.

Figure 2 b) is illustrative of an acoustically transmissive region 1339, adjacent to an acoustically non-transmissive region 1329 that is optically non-transmissive. Acoustic waves are dampened in the acoustically non-transmissive region 1329, and optical energy is absorbed at the boundary of the region 1329, which may produce an acoustic wavefront propagating to the acoustically transmissive region 1339. In an embodiment, the acoustically non-transmissive region 1329 comprises an optically absorbing agent 1328.

Figure 2 c) is illustrative of an acoustically transmissive optical distribution element 1360, adjacent to an acoustically non-transmissive region 1329 that is optically transmissive, where the boundary between the two regions is a rough or non-smooth pattern 1327 to reduce acoustic waves.

Figure 2 d) is illustrative of an acoustically transmissive optical distribution element 1360, adjacent to an acoustically non-transmissive region 1329, where a thin optically reflective material 1326 is between the element 1360 and the acoustically non-transmissive region 1329.

Figure 3 is an illustrative embodiment of an opto-acoustic probe with an acoustically transmissive optical distribution element 1260, showing a lengthwise cutaway view of the probe.

Figures 4 a) to l) are illustrative of numerous embodiments for an opto-acoustic probe 1300 with an acoustically transmissive optical distribution element 1360. The probe comprises a combined optical and acoustic port 1324. In an embodiment, the proximal surface 1314 of the acoustically transmissive optical distribution element 1360 is coupled to the surface of an acoustic transducer 1316. In an embodiment, optical energy is provided to an optical energy input 1325 on a surface of the optical distribution element 1360.

Figure 5 shows an embodiment of an opto-acoustic probe 1201 with and acoustically transmissive optical distribution element 1360 having an ergonomic form of a conventional ultrasound transducer. The figure shows a probe that is more narrow than other designs due to absence of light bars.

The methods and devices described herein provide illustrative examples of the subject invention including a probe for optoacoustic imaging having an acoustically transmissive optical distribution element 1360. The probe of the present invention may be adapted to be coupled with a volume 1370, to output light from its distal end and to have acoustic receivers 1310 that are adapted to receive acoustic signal from the coupled volume 1370. In an embodiment, the probe transmits light into the volume 1370 via an optical distribution element 1360. In an embodiment, the optical distribution element 1360 is made of light scattering material. In an embodiment, the optical distribution element 1360 comprises a reflective portion 1354 on its proximal end that is reflective. The reflective portion 1354 of the optical distribution element 1360 may be oriented to reflect light away from the acoustic receivers. The optical distribution element 1360 may be adapted to receive light from any non-reflective portion of the element 1360, which may include non-reflective portions of its proximal end, and its sides, and to permit light to exit its distal end. In an embodiment, the acoustic receivers 1310 may be acoustic transducers. In an embodiment, the acoustic receiver 1310 may be a single acoustic transducer.

In an embodiment, light from the optical distribution element floods the volume via a special element of material (i.e. a window) beyond the (coated) transducers 1210, 1310 to serve as a opto-acoustic window (a.k.a propagation element). The special element acts as an acoustically transmissive optical distribution element 1260, 1360 and diffuses and/or distributes the light within the element 1360 and permits acoustic waves to travel through the element 1360 as well. In an embodiment, a suitable material may be, or be similar to Plastisol (PVCP), which can have tuned optical and acoustic properties. In an embodiment, urethane may be a suitable material. The inventive probe described herein may be adapted for use in a multi-channel optoacoustic (OA) system, or single-channel OA unit, such as would be applicable to an EKG type OA pad or pulse-oximeter type unit. Moreover, the inventive probe described herein may be especially well adapted for use in a multi-wavelength multi-channel optoacoustic system. In an embodiment, light exits the optical distribution element where the optical distribution element is coupled to the volume over a fairly homogenous and broad area. In an embodiment, light enters the optical distribution element from a relatively small area, but exits the element generally towards the volume across a fairly homogenous and relatively large broad area. In an embodiment, when the optical distribution element is coupled to a volume the fluence caused by a given pulse of light entering the optical distribution element between two similar areas on the optical distribution element / volume interface, is substantially the same. In an embodiment, the probe 1300 is coupled to the volume 1370 using a coupling medium 1372 and light and sound pass through the coupling medium.

In an embodiment, a probe 1300 with a surface for delivering optical output may comprise: an acoustic transducer(s) 1310; an optical distribution element 1360 that is a combined optical scattering and acoustic propagation element between the tissue and the transducer (i.e. a window); the element 1360 having an optical output surface to distribute light to the tissue, the optical output surface configured to be placed proximate to the tissue, wherein the optical output surface of the optical distribution element 1360 serves as the primary light output delivery port of the probe, (thus the primary optical output of the combined element can be used in order to deliver light underneath the transducer 1310 instead of requiring the probe to have a light bar adjacent to the transducer); the element having optical properties such that it scatters light, but does not substantially absorb light (thereby permitting sufficient light to be passed through it to illuminate the tissue), in a manner similar to a diffuser (such as a ground glass diffuser); the element having at least one optical energy input 1325 surface to be fed optical input from an optical path 1330; one surface of the element coupled with the surface of the transducer 1316; the element such that it permits waves to be transmitted from the transducer to the tissue (assuming that the transducer is configured to transmit acoustically), the transmitted waves passing through the element as to minimize distortions, distortions including reflections; and the element permits waves to be received by the transducer from the tissue through the element, the received waves passing through the element as to minimize distortions, distortions including reflections. In an embodiment, the optical output surface is the distal surface 1312. In an embodiment, the optical output surface is a combined optical and acoustic port 1324.

In an embodiment, the acoustically transmissive optical distribution element 1360 of the probe may comprise polymer composition such as plastisol, PVC, urethane, especially when the density and speed of sound of the polymer closely match the acoustic impedance properties of the tissue to minimize interface reflections. In an embodiment, the element is made of a gelatin, which can be made opto-acoustically similar to a biological tissue. In an embodiment, the element is made of a material similar to a gelatin. In an embodiment, the element is made of a material similar to biological tissue. In an embodiment, the element is made of a material suitable for an opto-acoustic phantom.

In an embodiment, an opto-acoustic probe 1300 comprises an acoustic receiver 1310, an optical energy path 1330, and an exterior surface with a combined optical and acoustic port. In an embodiment, the probe 1300 comprises an acoustically transmissive optical distribution element 1360, comprising a distal surface 1312, and the distal surface 1312 is adapted to be coupled to a volume 1370 of a biological tissue to deliver optical energy to the volume 1370 and to exchange acoustic energy with the volume. In an embodiment, a coupling medium 1372 is used to acoustically and/or optically couple between the distal surface 1312 and the surface of the volume. In an embodiment, the probe 1300 also comprises a proximal surface 1314 proximate to the acoustic receiver 1310 to permit acoustic energy originating within the volume due to delivered optical energy to be detected by the acoustic receiver 1310 after the acoustic energy passes through the acoustically transmissive optical distribution element 1360. In an embodiment, the optical energy path 1330 of the probe 1300 is adapted to pass optical energy to one or more optical energy inputs 1325 of the optical distribution element 1360, and the optical distribution element 1360 distributes the optical energy from the one or more optical energy inputs 1325 to a combined acoustic and optical port 1324 on the distal surface 1312 and distributed optical energy exits the distal surface 1312 of the optical distribution element 1360. In an embodiment, the optical distribution element 1360 diffuses optical energy.

In an embodiment, the optical energy that exits the combined acoustic and optical port 1324 is distributed homogenously by the optical distribution element 1360. In an embodiment, the homogenous distribution of optical energy that exists the combined port 1324 has a constant optical energy as spatially distributed over the area of the combined port 1324. In an embodiment the spatially localized minimum and maximum optical energies exiting the combined port 1324 differ by no more than 10dB. In an embodiment, the minimum and maximum optical energies differ by no more than 3dB. In an embodiment, the variation in optical energy that exits the combined port 1324 is no greater than 6dB between any two positions located on the optical exit port. In an embodiment, the permitted maximum optical energy that exits the combined port 1324 is 20 mJ/cm$^2$. In an embodiment, the optical energy that exits the combined port 1324 is between 0.001 and 20 mJ/cm$^2$. In an embodiment, the optical energy output is greater than 20 mJ/cm$^2$. In an embodiment, the surface area of the combined port 1324 is between 0.001cm$^2$ and 1cm$^2$. In an embodiment, the surface area of the combined port 1324 is between 1cm$^2$ and 2cm$^2$. In an embodiment, the surface area of the combined port 1324 is between 1cm$^2$ and 10cm$^2$. In an embodiment, the surface area of the combined port 1324 is larger than 10cm$^2$.

In an embodiment, the optical distribution element 1360 comprises an optical scattering agent (e.g. titanium dioxide) for the purpose of scattering light and/or distributing light and/or diffusing light. In an embodiment, the optical energy distributed by the optical distribution element 1360 is distributed by scattering of optical energy by the scattering agent. In an embodiment, the concentration of the scattering agent may be controlled to achieve a homogenous distribution of light that exits the combined port 1324.

As shown in Figure 1 a) to c), the combined optical and acoustic port 1324 may be configured to have various shapes. In an embodiment, the combined port 1324 is surrounded by a housing 1303. In an embodiment, the housing 1303 has an exterior surface. In an embodiment, the housing comprises a shell(s) 1202, 1204. In an embodiment, the combined port 1324 is rectangular (Figure 1a and 1c). In an embodiment, the combined port 1324 is round (Figure 1b).

In an embodiment, plastisol may be mixed with a scattering agent such as titanium dioxide, or another material to affect the optical scattering properties of the element 1360 and cause the element 1360 to distribute light to a broader area than the area of the optical energy input 1325 to the element 1360.

In an embodiment, the proportion of scattering agent (e.g., titanium dioxide) to other materials can change as a function of distance from the optical input 1325 within the element (i.e., varies spatially), in such a manner as to improve the even uniform of distribution of light delivered to the volume. For example, a (lower) first concentration of a scattering agent may occur in one portion of element 1360 and a (higher) second concentration of the scattering agent may occur in another portion of the element. And this can be modelled by optical simulation (e.g. monte carlo) to attain a spatial distribution of scattering agent concentration (e.g. by varying the spatial distribution in the simulation model using optimization techniques) that will result in a desired optical output at the combined optical port 1324. In an embodiment, this results in a homogenous optical energy output from the port.

In an embodiment, the optical distribution element 1360 comprises the combined optical and acoustic port 1324, and the distal surface 1312 of the optical distribution element 1360 is coplanar with the exterior surface of the probe 1300. In an embodiment, combined optical and acoustic port 1324 comprises a protective layer 1352.

In a preferred embodiment, the acoustically transmissive optical distribution element 1360 is a solid-like material. In an embodiment, the distal surface 1312 and the proximal surface 1314 are parallel to each other. In an embodiment, the surfaces are aligned or overlap, meaning that an imagined line perpendicular to the (parallel) surfaces will mathematically intersect the both surfaces. In an embodiment, the solid-like material does not permit shear waves to travel. In certain circumstances, mode conversion created by shear waves can create unwanted signal, thus a solid-like material that does not substantially permit shear waves is desired. In an embodiment, a solid-like material is a plastisol, a gelatin, or other such material. In an embodiment, a solid-like material is a solid material. In an embodiment, a solid-like material is a flexible material.

In an embodiment, the probe 1300 is free of any optical exit ports (or light bars) for delivering opto-acoustic optical energy besides the combined optical and acoustic port 1324. If the probe 1330 is absent from any other optical exits ports for delivering opto-acoustic optical energy (besides the combined port 1324), this may permit the width of the probe to be more narrow than the case where the probe had light bars (or other optical exits), and thus the probe may have an ergonomic form of a conventional ultrasound probe (i.e. an ultrasound probe that is not an opto-acoustic probe). For example, when the probe has light bars adjacent to the transducer elements, the width of the light bars must be included in the total width of the probe, thus the probe width would in general be wider. However, when light bars are absent, and a combined optical and acoustic port 1324 is used instead, the total width of the probe 1300 may be thinner, and/or more ergonomic.

In certain circumstances, when delivering optical energy to the volume to illuminate the volume, it is beneficial to illuminate the volume directly beneath the transducer elements, rather than illuminating the volume adjacent to the transducer elements as would be the case when using a light bar adjacent to the transducer elements. In an embodiment, when the volume is illuminated directly beneath the transducer elements, optical energy is maximally delivered to the imaging plane (a plane intersecting the transducer elements perpendicular to the surface of the volume corresponding to the formed image). When light is delivered adjacent to the imaging plane, out-of-plane objects may be illuminated and produce undesired opto-acoustic return signal that is detected by the transducer elements. Thus, the combined optical and acoustic port 1324 may be used to reduce out-of-plane objects from occurring in opto-acoustic return signal. This can improve image quality, especially in the near-field.

In the embodiment shown in Figure 4l), the optical distribution element comprises an acoustic lens 1375, and the proximal surface 1376 of the optical distributive acoustic lens 1375 is coated, at least in part, with an optically reflective coating that is acoustically transmissive.

In the embodiment shown in Figure 4f), the optical energy path 1330 delivers optical energy to the optical distribution element 1360 from one or more side surfaces. The side surfaces are perpendicular to the distal surface 1312, and scattered optical energy exists the distal surface 1314 of the optical distribution element 1360 after optically scattering within the optical distribution element 1360.

In the embodiment shown in Figure 4h, the probe comprises multiple optical acoustic receiver elements 1311 and/or multiple optical energy inputs 1325 on a surface of the optical distribution element 1360.

In an embodiment, the optical distribution element 1360 has an acoustic impedance that gradually decreases (continuously or incrementally) from a first value to a second value, the first impedance value at the proximal end, the second impedance value at the distal end. This can improve acoustic signal transmission from the volume and can reduce reflections.

In an embodiment, the sides of the optical distribution element 1360 (e.g., the 4 side surfaces of a generally rectangular element) can couple to acoustic absorbing material (e.g., an isolator), the isolator 1321 having high acoustic absorption to dampen acoustic waves.

In an embodiment, the sides of the optical distribution element 1360 touching an isolator 1321 may be patterned 1327 to improve dampening of the acoustics. In an embodiment, an optically reflective coating 1326 is disposed between any sides or surfaces of the element 1360 (e.g., the 4 sides of the element 1360) and the absorbing material isolator 1321. In an embodiment the optically reflective coating 1326 may be disposed on the element 1360 or on the absorbing material 1321 or both. In an embodiment, the optically reflective material 1326 is a thin layer disposed on the sides of the element.

In an embodiment, the optical distribution element 1360 comprises a coating. In an embodiment, the distal surface 1312 comprises the coating 1352 and it is a hard material, to protect the element. In an embodiment the coating 1352 made of a hard material is thin. In an embodiment the optical distribution element coating 1352 is glass, and may be a thin layer of glass. In an embodiment, the coating 1352 is generally optically transparent. In an embodiment the surface coating 1352 is used to ensure that the distal surface thereof remains generally undeformed. In an embodiment the coating 1352 is used to ensure that the distal surface thereof remains generally planar.

In an embodiment, the optical distribution element is coated with an optically absorbing layer or feature that will produce an acoustic signal when exposed to a one or more wavelengths or spectra of light.

In an embodiment, the element is formed of a plurality of layers, and one or more of the layers are designed to be substantially more optically absorbing than the other layers, e.g., by adding small amounts of carbon black. In an embodiment, the element is formed of a plurality of layers, and each alternating layer is designed to be substantially more optically absorbing than the other layers, e.g., by adding small amounts of carbon black. In an embodiment, the element is formed of a plurality of layers, and at least one layer varies from the other in its optical absorption characteristics, and thus varies in the amount or type of acoustic signal that the layer will produce when exposed to a one or more wavelengths or spectra of light. In an embodiment, the element is formed of a plurality of layers, and a plurality of layers vary from the others in their optical absorption characteristics, and thus vary in the amount or type of acoustic signal that the layers will produce when exposed to a one or more wavelengths or spectra of light.

In an embodiment, the coating 1352 of the optical distribution element 1360 may comprise a material such as parylene for protection.

In an embodiment, the acoustically transmissive optical distribution element 1360 itself forms an acoustic lens for the acoustic receiver 1310. In an embodiment, an acoustic lens 1205 may be used between the optical distribution element 1360 and acoustic receiver 1310. In an embodiment, the optical distribution element 1360 fits around an acoustic lens 1205 that, at least in part, shapes the element in a manner to improve the signal reaching the acoustic receiver 1310. In an embodiment, the optical distribution element 1260 is shaped to fit snugly with an acoustic lens between the element and the acoustic receiver (e.g., having a cutaway or moulded portion being the negative of the lens). In an embodiment, the element 1360 comprises an acoustic lens, the acoustic lens portion of the element being made from a material having a different acoustic impedance from at least some other portions of the element.

In an embodiment, an acoustic lens comprises an optically transmissive material, wherein optical energy is passed from an optical path 1330 to an optical input port of the acoustic lens. In an embodiment, the acoustic lens acts as the acoustically transmissive optical distribution element 1360 and distributes light from its optical input port to exit a distal surface of the acoustic lens. Thus, the light passes through the acoustic lens from the optical input port to an optical exit port, acting as an optically distributive acoustic lens 1375. In an embodiment, a proximal surface 1376 of the acoustic lens is coated with an optically reflective coating, to prevent optical energy from reaching an acoustic receiver 1310 coupled to the proximal surface 1376 of the acoustic lens to prevent unwanted signal of the acoustic reciever. In an embodiment, the optically distributive acoustic lens 1375 comprises an optical scattering agent to scatter and/or distribute light within the acoustic lens. In an embodiment, the acoustic lens absorbs a portion of the optical energy creates an opto-acoustic wavefront that interferes with opto-acoustic return signal from the volume. In an embodiment, signal received by the acoustic receivers is mitigated by a processing unit.

In an embodiment, a distribution element is disposable, and can be easily removed (e.g., pops off) and easily replaced. In an embodiment, a plastisol opto-acoustic propagation layer is disposable, and can be easily removed (e.g., pops off) and easily replaced. In an embodiment, the disposable element comprises a gelatin.

In an embodiment, a sensor may be used to sense whether the disposable element and/or a plastisol opto-acoustic propagation layer is present, and/or has been properly installed on the probe.

In an embodiment, the acoustically transmissive optical distribution element 1360 includes an optically reflective coating 1354 between the element 1360 and the transducer 1310, to prevent the light from hitting the transducer and/or to reflect the light toward the volume. In an embodiment, the optically reflective coating 1354 is a metal, which may be gold, silver, brass, aluminum or another metal.

In an embodiment, the acoustically transmissive optical distribution element 1360 comprises multiple layers of different acoustic impedance values. Using multiple layers of different acoustic impedance values may assist with acoustic matching. In an embodiment, one or more of the multiple layers may be at least partially optically reflective. In an embodiment, at least some of multiple layers are light-scattering.

In certain circumstances, wave mode conversion may occur when the acoustically transmissive optical distribution element 1360 contains shear and longitudinal velocities. In certain circumstances, the layering of the acoustically transmissive optical distribution element 1360 and its coatings with different materials of different acoustic properties may serve to cancel, reduce or reflect shear wave components. This may include using anisotropic materials. In an embodiment, the element is designed to reduce acoustic propagation of shear waves. In an embodiment, the optical distribution element 1360 may comprise a layer or region of material that does not substantially transmit shear waves.

In an embodiment, at least portions of the optical path 1330 may extend into the acoustically transmissive optical distribution element. For example, the optical path 1330 may comprise optical fiber 1332. In an embodiment, the optical fiber 1332 in the optical path 1330 is in an optical cable or fiber bundle 1318. In an embodiment, at least some of the optical fibers 1333 of the optical path may extend into the acoustically transmissive optical distribution element 1360 rather than stopping at an interface outside the element. As a result, the optical fibers 1333 may be better able to deliver light into the element 1360. Moreover, in an embodiment, optical fibers 1333 within the element 1360 may be randomized, and/or may be zig and zagged around inside the element 1360. In an embodiment, the distal end or ends of one or more the optical fibers 1333 used in the light path are attached to an optical diffusor.

In an embodiment, optical fibers 1333 may poke out of holes in a plane parallel to the surface of the transducer, the fibers 1333 poking in to the interior of element 1360, the light being released into the interior of element 1360. In an embodiment, the distal end of one or more of the optical fibers making up the optical path 1330 may extend into the interior of the element 1360 through the proximal surface of the element 1314, thus permitting the light to be released from the light path into the interior of element. An optically diffusing fiber is a fiber that In an embodiment, an optically diffusing fiber may extend into the interior of the optical distribution element 1360, to release light into the interior of the element 1360. In an embodiment, light that exits an optically diffusing fiber may further scatter and/or diffuse as it passes through the interior of the optical distribution element 1360 towards the combined port 1324. In an embodiment the optical fibers 1333 and/or optically diffusing fiber enters or is proximate to an optically distributive acoustic lens 1375, to deliver energy to the acoustic lens 1375.

Figure 3 shows a lengthwise cutaway view of an embodiment of a probe 1200 with an acoustically transmissive optical distribution element 1260. The shells 1202, 1204 may be made from plastic or any other suitable material. The surfaces of the shells 1202, 1204 that may be exposed to light may be reflective or highly reflective and have low or very low optical and acoustic absorption. In an embodiment, flex circuit 1212 comprises a plurality of electrical traces (not shown) connecting cable connectors 1214 to an array of piezoelectric ultrasound transducer elements (not shown) forming ultrasound transducer 1210. In an embodiment, flex circuit 1212 is folded and wrapped around a backing 1211, and may be secured thereto using a bonding agent such as silicone. In an embodiment, a block 1213 is affixed to the backing 1211 opposite the array of piezoelectric ultrasound transducer elements. The cable connectors 1214 operatively connect the electrical traces, and thus, the ultrasound transducer 1210, to the electrical path. In an embodiment, the light path and electrical path are run through strain relief. In an embodiment, the optical path 1330 comprises light guides 1222. In an embodiment, the light guides are used to support and/or position optical fibers therewithin to provide structural support and/or to provide repeatable illumination.

In an embodiment, an acoustic lens 1205 is located in close proximity to, or in contact with the ultrasound transducer 1210. In an embodiment, the acoustic lens 1205 is an optically distributive acoustic lens 1375 (configuration not shown here), and receives optical energy from light guides 1222. In an embodiment, the acoustic lens is coupled to an acoustically transmissive optical distribution element 1260. In an embodiment, the distal surface 1224 of the optical distribution element 1260 is a combined acoustic and optical port 1324. The acoustic lens 1205 may comprise a silicon rubber, such as a room temperature vulcanization (RTV) silicon rubber. In an embodiment, the ultrasound transducer 1210 is secured behind the acoustic lens 1205 using a suitable adhesive such as silicone. The transducer assembly 1215, thus, may comprise the acoustic lens 1205, ultrasound transducer 1210, the flex circuit 1212 and its cable connectors 1214, the backing 1211, and a block (not shown). In an embodiment, the backing 1211 or block can be used to affix or secure the transducer assembly 1215 to other components.

In an embodiment, the RTV silicon rubber forming the acoustic lens 1205 may be doped with TiO2. In an embodiment, the RTV silicon rubber forming the acoustic lens 1205 may be doped with approximately 4% TiO2. In an embodiment, the RTV silicon rubber forming the acoustic lens 1205 may be doped with between 0.001% and 4% TiO2. In an embodiment, the outer surface 1206 of the acoustic lens 1205 may additionally be, or alternatively be, coated with a thin layer of metal such as brass, aluminum, copper or gold. In an embodiment, the outer surface 1206 of the acoustic lens 1205 may first coated with parylene, then coated with nickel, then coated with gold, and finally, again, coated with parylene. In an embodiment, the portions of the acoustic lens 1205 having a parylene coating edge are adapted to be mechanically secured against other components to prevent curling or peeling. In an embodiment, substantially the entire outer surface 1206 of the acoustic lens 1205, is coated with continuous layers of parylene, then nickel, then gold and then parylene again. In an embodiment, substantially the entire outer surface of the acoustic lens 1205 (but not its underside) may be coated with a continuous layer as described. Portions of the transducer assembly 1215 behind the acoustic lens 1205 may be surrounded, at least in part, by a reflective material, which may also serve as an electromagnetic shield.

Isolators 1220 physically separate the transducer assembly 1215 from other probe components, including optical distribution element 1260, light guides 1222, and in an embodiment, diffusers, which may be, among other choices, holographic diffusers or ground or frosted glass beam expanders. In an embodiment, isolators 1220 are formed in a manner to aid in location and/or securing of optical distribution element 1260, diffusers and/or the acoustic lens 1105. In an embodiment, isolators 1220 comprise ridges or detents for to aid in location and/or securing of optical distribution element 1260, diffusers and/or the lens 1205. Additional acoustic isolators 1221 may also be positioned between the acoustically transmissive optical distribution element 1260 and the probe shells 1202, 1204.

The isolators 1220, 1221 are made from materials that reduce the optoacoustic response to light generated by the light subsystem which is ultimately transmitted to the transducer 1210 during sampling. In an embodiment, the isolators 1220, 1221 are fabricated from a material that absorbs light (or reflects light) and substantially prevents light from reaching the transducer assembly 1215, but also dampens transmission of acoustic (e.g., mechanical) response to the light it has absorbed as well as the acoustic energy of surrounding components. In an embodiment, the isolators 1220 are positioned so as to be substantially in the path of mechanical energy - such as any optoacoustic response, that originates with other components (e.g., the optical distribution element 1260, or diffusers) - that may reach the transducers 1210 during an acoustic sampling process. In an embodiment, when assembled, the isolator 1220 surrounds at least a substantial portion of the acoustic transducer assembly 1215. In an embodiment, when assembled, the isolator 1220 completely surrounds the acoustic transducer assembly 1215. By surrounding the transducer assembly 1215 with the isolators 1220 and fabricating the isolators 1220 from materials having the foregoing characteristics, the amount of mechanical or acoustic energy reaching the transducer 1210 during sampling is mitigated.

In an embodiment, the isolator 1220 is fabricated to fit snugly against the flex circuit 1212 when it is assembled. In an embodiment, a thin layer of glue or other adhesive may be used to secure the isolator 1220 in relation to the flex circuit 1212, and thus, in relation to the transducer assembly 1215. In an embodiment, the fit is not snug, and a gap between the isolator 1220 and the flex circuit 1212, and/or the backing 1211 is filled, at least partially, with a glue or adhesive. In an embodiment, the isolators 1220 are fabricated from materials that will absorb that energy. In an embodiment, the material used to fabricate the isolators 1220, 1221 is a compound made from silicone rubber and microspheres.

In an embodiment, an isolator 1320, 1321, 1220, or 1221 is fabricated from a flexible carrier, and microbubbles. As used herein, the term microbubbles includes microspheres, low density particles or air bubbles. In an embodiment, an isolator 1320, 1321, 1220, or 1221 may be fabricated from components in the following proportions: 22 g flexible material as a carrier; and from about 10% to 80% microspheres by volume. In an embodiment, an isolator 1320, 1321, 1220, or 1221 comprises at least a small amount of an optical absorbing agent (i.e. coloring), but not so much that it thickens past mix-ability. In an embodiment, an isolator 1320, 1321, 1220, or 1221 may be fabricated from components in the following proportions: 22 g flexible material as a carrier; but not so much that it thickens past mix-ability; and about 10% to 80% air by volume, the air occurring in small bubbles. In an embodiment, an isolator 1320, 1321, 1220, or 1221 may be fabricated from components in the following proportions: 22 g flexible material as a carrier and about 10% to 80% low density material particles - as compared to the flexible carrier. Although several of the foregoing proportions are given using 22 g of flexible carrier, that number is only given as an illustration. What is important are the proportional ranges of the materials used, not that it is made in batches of a specific size.

In an embodiment, the microspheres may have shells made from phenolic, acrylic, glass, or any other material that will create gaseous bubbles in the mixture. In an embodiment, the microspheres are small individual hollow spheres. As used herein the term sphere (e.g., microsphere), is not intended to define a particular shape, e.g., a round shape, but rather, is used to describe a void or bubble - thus, a phenolic microsphere defines a phenolic shell surrounding a gaseous void which could be cubic, spherical or other shapes. In an embodiment, air bubbles or a low density particles may be used instead of, or in addition to, the microspheres as microbubbles. In an embodiment, the microspheres, low density particles or air bubbles may range in size from about 10 to about 250 microns. In an embodiment, the microspheres, low density particles or air bubbles may range in size from about 50 to about 100 microns. In an embodiment, the isolator 1320, 1321, 1220, or 1221 is formed from two or more parts. In an embodiment, the isolator 1320, 1321, 1220, or 1221 is formed in two substantially identical halves.

In an embodiment, the silicon rubber compound may be a two part silicon rubber compound that can cure at room temperature. The flexible carrier may be a silicone rubber compound, or other rubber compound such as a high temperature cured rubber compound. In an embodiment, the flexible material may be any plastic material that can be molded or otherwise formed into the desired shape after being compounded with microspheres, low density particles and/or air bubbles and color ingredients. In an embodiment, the flexible carrier may be a plastisol or a gelatin. In an embodiment, portions of the acoustically transmissive optical distribution element 1360, 1260 may be filled with microspheres to create acoustically non-transmissive portions 1329 that block sound waves. In an embodiment, acoustically non-transmissive portions 1329 are abutted to acoustically transmissive portions of the optical distribution element 1360. In an embodiment where microbubbles are optically transmissive (Figure 2a) light may transmit from the acoustically transmissive optical distribution element 1360 into an adjacent acoustically non-transmissive portion 1329. In an embodiment, (Figure 2b) the acoustically non-transmissive portion 1329 may be filled with optically absorbing particles 1328 causing light to be blocked from traversing the acoustically non-transmissive portion (i.e. an acoustically non-transmissive and optically non-transmissive portion). In an embodiment, the optically absorbing particles are optically absorbing microbubbles. In an embodiment, the optically absorbing particles are particles of a light absorbing agent or a coloring. In an embodiment, when the optically absorbing particles absorb light and acoustic wave may be produced. In an embodiment, the acoustically non-transmissive portion 1329 blocks an acoustic wave generated by optically absorbing particles 1328. In an embodiment, when the optically absorbing particles 1328 of the acoustically non-transmissive portion 1329 block light, light is only absorbed at the boundary of the acoustically non-transmissive region 1329. Thus, an acoustic wave is blocked from passing through the acoustically non-transmissive portion, however an acoustic wave may still travel from the optically absorbing surface to adjacent acoustically transmissive materials. In an embodiment, the acoustically non-transmissive portion is an isolator 1320, 1321. The light absorbing agent (coloring) may be carbon black, or any other suitable coloring, including ink or dye, that will impart a dark, light-absorbing characteristic to the mixed compound. In an embodiment, the boundary of the acoustically non-transmissive region 1329 adjacent to the optical distribution may be patterned 1327 to have a rough or non-flat surface to acoustically scatter and/or reduce acoustic waves (Figure 2c). In an embodiment, an optically reflective material or optically reflective coating 1326 is placed between an acoustically non-transmissive region and an acoustically transmissive region (Figure 2d).

In an embodiment, the sides of the optical distribution element 1360 may be acoustically reflective. In an embodiment, the sides of the optical distribution element 1360 will be acoustically reflective if the side surfaces are adjacent to an air gap. In an embodiment, received acoustic waves reflected of the side surfaces of optical distribution element 1360 originating from the volume passing through the element 1360 that are received by the acoustic receivers 1310, may be useful in reconstruction to improve limited-view performance. In an embodiment, waves reflected of the side surfaces of the element 1360 contain information direct acoustic return that is not otherwise accessible to transducer elements oriented normal to the surface of the volume due to the directivity of the elements. In an embodiment, a reconstruction (or a simulation) taking into account reflections of the side surfaces of the element 1360 may improve visibility of the volume. In an embodiment, the element is acoustically simulated and/or modelled as a wave-guide.

In an embodiment, the following steps can be used to fabricate the isolators 1320, 1321, 1220, or 1221. A mold may be prepared by applying thereto a thin release layer, such as a petroleum jelly. The ingredients are carefully measured and mixed until a uniform consistency is reached. Note care should be exercised in mixing because excessive mixing speed may entrap air in the mixture. The mixture is then placed into a mold appropriately shaped to form the isolator 1320, 1321, 1220, or 1221 (or parts thereof). In an embodiment, an instrument is used to work the mixture into the corners of the mold. The mold is closed and pressed, with excess permitted to exit through vent holes. The mixture is then permitted to cure. Once cured, the casted part may be removed from the mold and cleaned to remove excess material, as is common, with a razor blade or other instrument(s). The cleaned parts may be washed with soap and water and wiped with alcohol to remove grease and/or dirt.

In an embodiment, portions of the fabricated part are coated with a reflective or highly reflective material such as gold or brass powder. In an embodiment, reflective gold coating may be used. In an embodiment, to coat the part, acrylic can be added drop-wise to a small amount of gold, brass or other reflective material until a suitable gold paint is achieved. In an embodiment, any reflective paint, e.g., gold colored paint, may be used. In an embodiment, to avoid coating portions of the isolators 1320, 1321, 1220, or 1221 if required, surfaces of the isolators 1320, 1321, 1220, or 1221 may be taped, such as with Teflon tape. In an embodiment, gold paint is painted on the front and side of the isolators 1320, 1321, 1220, or 1221, the sides that will contact optical distribution element 1360, 1260, glass, diffuser and/or other components, In an embodiment, an isolator 1320, 1321, 1220, or 1221 may be shaped to hold the element 1360 in place. Figure 5 shows an embodiment of an opto-acoustic probe 1201 with an ergonomic form of a conventional ultrasound probe. The probe 1201 comprises an acoustically transmissive optical distribution element 1260. In an embodiment, the sides 1226 of the element 1260 have an optically reflective coating. Light is emitted from light guides 1222. In an embodiment, the light guides are designed to house optical fibers. Light exits from the optical pathway exit 1223 and is input to the optical distribution element 1260, where light diffuses and scatters, and exits the combined acoustic and optical port 1224. In an embodiment, an isolator 1221 may be positioned between the sides 1226 of element 1260 and the shells 1202, 1204. In an embodiment, the shells are acoustically absorbing.

In an embodiment, the optical pathway 1330 of Figure 4 comprises an optical pathway exit port 1323 that passes optical energy to an optical energy input 1325 on a surface of the optical distribution element 1360. In an embodiment, the optical pathway exit port 1323 is coated with an optical and/or acoustic coating. In an embodiment, the optical pathway exit coating 1350 improves optical transmission to the optical distribution element 1360. In an embodiment, the signal path 1313 carries optical and/or electrical signals and/or energy to the probe. In an embodiment, the signal path 1313 caries electrical signals from the acoustic receivers 1310 and/or the transducer assembly 1315, 1215. In an embodiment, the signal path 1313 is a combined optical and electrical signal path 1317 (e.g. a cable comprising an optical energy portion and an electrical signal portion). In an embodiment, the optical pathway 1330 comprises an optical cable. In an embodiment, the optical pathway 1330 comprises an optical signal path 1319. In an embodiment, optical energy is produced within the probe (e.g. a LED or laser diode) and thus an optical cable connecting to the probe is not required.

In an embodiment, the optical distribution element 1360 absorbs at least a portion of the optical energy is receives and to produces an acoustic wave. In an embodiment, the acoustic wave creates a secondary acoustic return that interferes with a direct acoustic return component originating from the volume.

**Embodiments With Component Separation**

In an embodiment, the opto-acoustic probe is connected to an opto-acoustic system comprising a processing unit adapted to separate the secondary acoustic return from the direct acoustic return component using a component separation algorithm.

In an embodiment, an algorithm to separate an unwanted signal generated by the optical distribution element 1360 from a direct acoustic return is used. In an embodiment, an algorithm and/or filter to mitigate an unwanted signal generated by the optical distribution element is used (e.g. a bandpass filter, an interframe persistent artifact removal). In an embodiment, an image based on the separated direct acoustic return component and/or mitigated direct acoustic return is generated and displayed to a display. Methods for separating direct acoustic return from secondary acoustic return that are described herein are applicable to removing unwanted signal that may be produced by the acoustically transmissive optical distribution element 1360. It will be apparent to one skilled in the art that when such processing is not used, designing a probe that uses an acoustically transmissive optical distribution element 1360 may require more design limitations, as certain embodiments could produce strong unwanted signal components (e.g. shear waves, secondary acoustic return, reflections, reverberations, aberration); however, with algorithms such as those described herein, using an embodiment of acoustically transmissive optical distribution element 1360 that results in potentially some unwanted distortion to an unprocessed signal becomes a practical option by using processing to prevent the unwanted distortion from occurring in image output. Thus, the choice of materials that can be used practically for the element 1360 is thus not limited to materials where distortions are low. In a preferred embodiment, however, distortions from the element 1360 are in fact low.

Opto-acoustic systems

As is known in the art, opto-acoustic systems may take many forms. Generally, an opto-acoustic (or photoacoustic) system acquires an acoustic signal that is created as a result of electromagnetic energy being absorbed by a material. While other types of electromagnetic energy may be used, opto-acoustics is generally associated with the use of electromagnetic energy in the form of light, which light may be in the visible or near infrared spectrum. Thus, an opto-acoustic system has at least one source of electromagnetic energy and a receiver that acquires an acoustic signal that is created as a result of electromagnetic energy being absorbed by a material.

Certain embodiments of an opto-acoustic system are discussed in U.S. Patent Application US20130281819, entitled "Noise Suppression in an Optoacoustic System". The identified patent application describes an embodiment of an opto-acoustic system comprising a plurality of light sources that are an opto-acoustic system capable of outputting pulses of light (at differing predominant wavelengths) to a probe via a light path. Light exits the probe through one or more optical exit ports at the distal end, and the one or more ports may have an optical window across the port. A receiver also at the distal end of the probe is used to sample an acoustic signal. In an embodiment, the receiver may be a multi-channel transducer array which may be used to sample an opto-acoustic return signal at a sampling rate. In an embodiment, the receiver may sample at 31.25 Mhz for a duration of about 65 $\mu$s. The samples are stored as a sinogram. In operation, after the distal end of the probe is brought into proximity with a volume to be imaged, the opto-acoustic system as described above may pulse one of its light sources and then sample an acoustic signal. Generally, as discussed in the prior patent application, the predominant wavelengths of the light sources may be selected to be compatible (i.e., highly absorbed) by the features sought to be identified by opto-acoustic imaging.

Although the foregoing describes specific embodiments of an opto-acoustic system, it is presented for illustration only, and the discussion below is not so limited. As discussed in more detail below, portions of the disclosure herein are applicable to an opto-acoustic system having fewer or more light sources, e.g., one light source, or three or more light sources, each of which may have a different predominant wavelength. As will also be apparent, it is also applicable to an opto-acoustic system having multiple light sources capable of producing a pulse at the same wavelength in close succession, or to having one or more light sources (each operating at a different wavelength), and one or more of them being capable of producing pulses in close succession to each other. Moreover, although the foregoing describes embodiments of an opto-acoustic system having transducers capable of outputting ultrasound energy, as discussed in more detail below, such transducers may be unnecessary, and in an embodiment, acoustic receivers will suffice in their stead.

As used herein, the term sinogram refers to sampled data (or processed sampled data) corresponding to a specific time period which may closely follow after one or more light events, or may coincide with one or more light events, or both. Where sinograms are referred to as long sinograms or short sinograms, these generally refer to a sampled acoustic signal from two different light events, each corresponding to a different wavelength of light, the term short sinogram thus refers to the sinogram corresponding to the shorter wavelength of light generating a light event, and the term long sinogram refers to the sinogram corresponding to the longer wavelength of light generating a light event. Because fewer or more than two wavelengths may be used, the use of the terms short and long wavelength are intended to embody the extended context of a system with an arbitrary number of wavelengths.

For illustration throughout, but not by way of limitation, and except where the context reveals otherwise, a sinogram represents a finite length sample of acoustic signal, sampled from an array of receivers. As an example, in an embodiment, a sinogram may represent a sample of 128 channels of a receiver for 65 $\mu$s at 31.25 Mhz. While the discussion below may relate to this example sinogram, the specific length, resolution or channel count are flexible, and substantial variation will be apparent to one of skill in the art without departing from the scope of the present disclosure. Moreover, the examples discussed below generally reflect a linear array of acoustic receivers, however, neither the organization of the receivers nor its number of channels are meant by way of limitation, and substantial variation will be apparent to one of skill in the art without departing from the scope of the present disclosure.

Sinogram Components

As discussed above, a sinogram may contain, essentially, a sampled recording of acoustic activity occurring over a period of time. Generally speaking, the sinogram is recorded to capture acoustic activity that occurs in response to one or more light events, although, as noted above, the light event(s) may occur shortly before, or during the sampling period, or both. The acoustic activity captured (or intended to be captured) in the sinogram includes the opto-acoustic response, that is, the acoustic signal that is created as a result of electromagnetic energy being absorbed by a material.

For the purposes of discussion of the basic principals involved, as an illustration, a probe-type opto-acoustic system such as described above may be used. The probe is brought in close proximity with a volume of tissue (which is not particularly homogenous), and a sinogram may be created by sampling the opto-acoustic response to one or more light events (from one or more light sources) occurring either shortly before or during the sampling period. Thus, the resulting sinogram contains a record of the acoustic activity during the sampling period. The acoustic activity during the sampling period, however, may contain information that is not related to the one or more light events created for the purpose of making the sinogram. Such information will be referred to as noise for the purposes of this section. Thus, for these purposes, the sinogram comprises noise and opto-acoustic response.

The opto-acoustic response includes acoustic signals that result from the release of thermo-elastic stress confinement- such acoustic signals may originate from one or more optical targets within the volume in response to the light event(s). Some of the opto-acoustic response in the sinogram propagated through the volume essentially directly to the receivers, while some is reflected or otherwise scattered within the volume before arriving at the receivers. The portion of the opto-acoustic response in the sinogram which propagates through the volume essentially directly to the receivers- that is, without substantial reflection or scattering off an acoustic target- is referred to herein as the "Direct Acoustic Return" or "DAR." In addition to noise and DAR, other acoustic signals that reach the receiver and originate in the volume may be caused by a variety of phenomena. The portion of the opto-acoustic response in the sinogram which propagated through the volume but were substantially reflected or scattered before arriving at the receiver- including signals that reach the receiver and originate in the volume, but are the reflected or scattered portions of the wavefronts causing the DAR signal - are referred to herein as the "Secondary Acoustic Return" or "SAR." Since an entire volume is susceptible to some level of opto-acoustic response, all discontinuities in the system (which for the purpose of this section includes the volume and the probe) may create reflections or secondary scattering that occur at the boundaries. For the purposes herein, these scattered and reflected signals, to the extent they reach the receiver, are also deemed SAR. In addition to DAR, SAR and noise, the sinogram may comprise other signals, including, without limitation, surface waves, shear waves and other signals that may be caused by the light event(s) originating within or external to the volume.

In some circumstances, acoustic targets in the volume may slightly deflect an acoustic wave originating from an optical target such that most of the energy of the wave continues to propagate along a slightly deflected path. In these circumstances, the wave originating from the optical target may still be considered DAR (especially where the path deviation is small or signal arrival time deviations are accounted for). This is to say that in some circumstances, e.g., in non-homogenous media, the direct acoustic response may follow a curve rather than a straight line, or the acoustic wave may travel a path that is deflected at certain acoustic boundaries within the volume or coupling medium. In other circumstances, for example, where the speed of sound of the volume or surroundings is not constant or homogenous, a DAR wavefront travelling from an optical target to two acoustic receivers each positioned equal distances away from the target may be reached by portions of the wavefront at different times. Using these general guidelines and the discussion presented below, the difference between DAR and SAR will be apparent to one skilled in the art.

In embodiment that include component separation, novel methods and apparatuses are used for processing opto-acoustic data to identify, separate or remove unwanted components from the sinogram, and thereby improve the clarity of an opto-acoustic image based thereon. For example, there is a discussion concerning a novel means of removing SAR components that are commonly referred to as backscatter. Also present in the Component Separation section is a disclosure of a novel method and system to identify, separate and remove the effect of surface waves from the sinogram. The Component Separation section also discusses novel methods and apparatus to separate information from multiple light events (at different predominant wavelengths) that are present in the sinogram. The Component Separation section also discusses novel processes and systems to improve the signal-to-noise ratio, among other things, using information from multiple light events (at a single predominant wavelength) that are present in the sinogram. And the Component Separation section discusses a novel method and device for using separated SAR components as functional information and potentially to create functional imagery. Certain embodiments of an opto-acoustic probe that has features which may be useful for application in component separation are discussed in WO2013056089 entitled "System and Method for Acquiring Optoacoustic Data and Producing Parametric Maps Thereof".

Also described in this disclosure are coded probe embodiments, which expand on the discussion of removing SAR components, by using the natural path of the photons emitted by a light event to illuminate specific targets external to the volume, and thereby can create known, or expected, SAR components, and/or amplify the existing SAR. In some embodiments, specific features and/or properties of the probe itself are provided and to create known, or expected, SAR components, and/or amplify the exiting SAR. The thus-injected SAR components can be used to aid in identification and removal of SAR components, and may further enhance the ability to separate SAR components for use as functional information. The specific targets external to the volume can be encoded to produce specific responses, including differing amplitude and/or frequency responses, and may further be designed to be more or less responsive to one of the several light sources available in a multiple light source embodiment.

In an embodiment, the acoustic receivers may detect waves caused by the specific targets. In an embodiment, the acoustic receivers may detect surface or shear waves caused by the specific targets. In an embodiment, the method and apparatus can be part of a combined opto-acoustic probe.

DAR vs. SAR Separation

Figure 6 shows a block diagram of an embodiment of a Component Separation System. The system in this embodiment includes an energy source, a receiver, a processing subsystem, an output device and a storage device. In an embodiment, the energy source comprises at least one light source for delivering light energy to a volume of tissue and the receiver comprises a transducer array for receiving a resulting acoustic signal. The processing subsystem processes the acoustic signal to separate a DAR component from a SAR component of the acoustic signal, and the output and/or storage device presents and/or stores information about the DAR component, the SAR component, or both. It will be apparent to one skilled in the art that, in an embodiment, other sources of electromagnetic energy may be used in place of a light source. It will also be apparent to one skilled in the art that, in an embodiment, a single receiver or group of receivers may be used in in place of a transducer array. Each of these components is described in more detail below along with other possible components.

In an embodiment, the system is used to isolate and/or remove from an acoustic signal or spatial representation one or more artifacts caused by one or more acoustic wavefronts. As discussed above, acoustic wavefronts can be caused by various sources.

In an example, one or more acoustic wavefronts can reflect (or scatter) off one or more acoustically reflective targets in a given volume causing a SAR component of the acoustic signal. Figure 7 shows two images reconstructed from an acoustic signal received from a given volume. The top image is an ultrasound image, while the bottom image is an opto-acoustic image overlayed on an ultrasound image. The effective depth of the images has been doubled beyond the applicable ultrasound depth to demonstrate the opto-acoustic artifact. The region 210 in the top image represents rib tissue and beneath it is lung tissue in the given volume. It is believed that the wave interference in the bottom image is caused by reflection 220 of an acoustic wavefront originating at the surface off of the lung or rib tissue. The lung or rib tissue and artifacts shown here are merely examples. Acoustic wavefronts may reflect or scatter off of other acoustically reflective targets, including parenchymal tissue, in a volume causing similar or other artifacts. In an embodiment, one or more of the processes or systems described herein can be used to isolate and/or remove such artifacts from signals and/or spatial representations of the volume.

In an embodiment, the system comprises at least one light (or other energy) source configured to deliver electromagnetic energy to a volume of tissue such that when the electromagnetic energy is delivered an acoustic signal is detectable with at least two components: 1) a DAR component; and 2) a SAR component. The DAR component generally results from temporal stress confinement within one or more electromagnetically absorbent targets in the volume. The SAR component generally results from the incidence of at least one acoustic wavefront on one or more acoustically reflective (i.e., acoustically scattering) targets in the volume. The electromagnetically absorbent targets may also be targets of some acoustic backscatter. Correspondingly, the acoustically reflective targets may also be targets of some electromagnetic energy absorption. Thus, the sets of acoustically reflective targets and electromagnetically absorbent targets need not be mutually exclusive, and may overlap in whole or in part. In an embodiment, the DAR and/or SAR signals are ultrasound signals. In an embodiment discussed in more detail herein, the electromagnetic energy is light energy and the DAR signal is an opto-acoustic return signal. In an embodiment, the electromagnetic energy is energy from part of the RF spectrum, that is, other than light energy. As will be appreciated by one skilled in the art, many, and potentially all portions of the RF spectrum, may cause a DAR signal, and thus, the invention disclosed herein is not limited to use in connection with the visible light energy portion, or even just the light energy portion of the RF spectrum.

In an embodiment, the system includes at least one acoustic receiver configured to receive at least a portion of the DAR signal component and a least a portion of the SAR signal component. In an embodiment, the acoustic receiver may include transducers, which may be located at the distal end of an opto-acoustic probe. In an embodiment, the DAR signal and the SAR signal both reach the acoustic receiver during a single sampling cycle, e.g., a 65 $\mu$s of sampling at 31.25 Mhz as described above. At least a portion of the SAR signal may be caused by acoustically reflective targets backscattering acoustic energy from an incident wavefront produced at the surface in response to a light event, as described in more detail below. Because the electromagnetic energy propagates through the volume faster than the acoustic wavefront, with respect to a given target, there is generally a delay of the reception of the SAR signal in comparison to the DAR signal. Thus, under some circumstances, the DAR signal and the SAR signal from a specific target reach the receiver at different times. Under some circumstances, however, the DAR signal and the SAR signal may, at least in part, reach the receiver simultaneously (e.g., when the target is touching the receiver). In an exemplary embodiment, the electromagnetic energy is light energy, which propagates through the volume at or near the speed of light (and in any event, at a speed much faster than the acoustic wavefront) while the acoustic wavefront propagates through the volume at a much slower speed, which speed is nearer the speed of sound (e.g., the speed of sound in tissue). In such an exemplary embodiment, where the acoustic receiver and the source of the electromagnetic energy are at about the same distance from the electromagnetically absorbent and the acoustically reflective targets, it can be assumed that the DAR signal reaches the receiver about twice as fast as the SAR signal from a given target.

In an embodiment, the acoustic receiver may be an array of acoustic receivers. In an embodiment, the receivers in the array of acoustic receivers are transducers, and may be piezoelectric transducers. In an embodiment, the acoustic receiver comprises at least one transducer that is capable of generating an acoustic wavefront that propagate through the volume. In an embodiment, reflective mode imaging is used, where the receivers are proximate to the energy source, which is typically the case when receivers and energy source are both on a handheld probe. In an embodiment, the electromagnetic energy is delivered via a probe and a receiver may be positioned on the probe, and in particular, it may be positioned on the distal end of the probe (i.e., the end closest to the volume). In an embodiment, where, for example, a transmission mode is utilized, a receiver may be positioned at a location near or adjacent to the volume, but not proximate the source of the electromagnetic energy delivery. In transmission mode, the receiver is commonly placed on the opposite side of the volume from the electromagnetic energy source. When an incident wavefront originates substantially opposite the volume to the receiver, an acoustic scattering target in the volume may predominantly cause an acoustic reflection that does not reach the receiver, but rather the scattering may affect the acoustic transmission of the incident wavefront that is measured by the receiver. Since, acoustically scattering targets may reflect and transmit acoustic wavefronts according to a relationship, an acoustically reflective target may also be considered as an acoustically transmissive target and vice versa. The reflective scattering strength of an acoustically reflective target does not always equal its transmissive scattering strength. In an embodiment, no distinction is made between an acoustically scattering target, and an acoustically reflecting target or an acoustically transmissive target. In an embodiment, a system is designed to provide stronger analysis of signals resulting from reflections of acoustic targets rather than the signals resulting from an acoustically scattering target or an acoustically transmissive target. For example, when wavefronts originating from the surface of a handheld probe reach a target, the reflected wavefront from the target may be directed back towards the probe, but the transmitted part of the wavefront may keep going and may not reach an acoustic receiver on the probe. Hence, in some circumstances, some transmitted or reflected scattering reflections may not be received by receivers or analyzed by the processing subsystem described next.

With further reference to Figure 6, in an embodiment, a processing subsystem is adapted to analyze the acoustic signals to obtain information regarding electromagnetically absorbent and/or acoustically reflective targets in the volume. In an embodiment, the processing subsystem analyzes the acoustic signals (e.g., in sinograms) to produce a spatial representation of the targets in the volume. In an embodiment, the subsystem uses a time delay between the reception of the DAR signal and the SAR signal to better analyze the signals. In an embodiment, the system separates the DAR signal (or spatial representation thereof) and the SAR signal (or spatial representation thereof) and processes them differently based on the time delay and/or other parameters.

In an embodiment, the processing subsystem comprises: 1) a reconstruction module capable of analyzing acoustic signals (such as the DAR signal and the SAR signal discussed above) to produce estimated spatial representations of targets in a volume (such as the electromagnetically absorbent targets and the acoustically reflective targets discussed above); and 2) a simulation module capable of analyzing spatial representations of targets in a given volume (such as the estimated spatial representations produced by the reconstruction module) and generating acoustic signals that might be produced by applying electromagnetic energy to the given volume. In an embodiment, the reconstruction and simulation modules perform adjoint operations: the reconstruction module obtaining acoustic signals and producing spatial representations; and the simulation module obtaining spatial representations (such as those produced by the reconstruction module) and producing (e.g., back-projecting) acoustic signals that might be produced when electromagnetic energy is applied to a volume with the given spatial representations. In an embodiment, the simulation module performs a forward projection. In an embodiment, the simulation module further preforms additional processing which may include accounting for in-homogeneity, propagation delay, denoising, or other additional processing. In an embodiment, the forward projection may use a system transfer matrix. In an embodiment, the reconstruction module performs a backward projection. In an embodiment, the backward projection may be the Hermitian adjoint of the forward projection. In an embodiment, the reconstruction module further performs additional processing which may include accounting for in-homogeneity, propagation delay, adaptive filtering, or other additional processing. The spatial representations and acoustic signals can be passed, received, or stored in any convenient format, and various formats for the same will be apparent to one of skill in the art in view of this disclosure. In an embodiment, the spatial representations are passed, received, or stored as an array of pixels, a bit map, or other image format. In an embodiment, three or higher dimensional representations may be passed, received, or stored. In an embodiment, the acoustic signals may be passed, received, or stored as sinograms. Other formats and representations are known in the art and can be used in connection with the disclosures herein, such other formats and representations including, without limitation, transformed domains such as wavelet or similar transformations, dictionaries, or a representation basis, which may improve performance. Accordingly, the spatial representation can include wavelet representation of the spatial domain or other such applied transformation to the spatial domain, where applicable. In an embodiment, during various stages of processing, a representation may switch to and from a transformed representation represented in different basis such that the transformation substantially preserves all of the data (e.g. a wavelet transformation applied to a spatial representation). Such switches may or may not be fundamental to the performance of the processing (e.g., performing thresholding on a sparse representation); however, the stages of processing where transformation does occur may vary between implementations. Hence, in an embodiment, such transformations may be inserted in various stages of processing. The correctness and applicability of applying such transformations should be apparent to one skilled in the art.

In an embodiment, the spatial representation may be a 2D array representing a 2D slice of the volume. In an embodiment, the spatial representation may be a 3D array representing a 3D region of the volume. In an embodiment, the spatial representation may be a wavelet representation of a 2D slice or 3D region of the volume. In an embodiment, when a 1D array of transducers is used to record sinogram measurements and a 3D spatial representation of the volume is used, iterative minimization techniques (such as those described below), may be applicable to determining out-of-plane structures. Similarly, application of iterative minimization techniques may be advantageous when a 1.5D or 2D array of transducers is used. The choice of the basis for the 3D spatial representation (e.g., wavelet) can affect processing speed and/or image quality performance. Hence, in an embodiment, the steps of 1) iteratively reconstructing a 3D representation of the volume, then 2) extracting a 2D slice from the 3D representation, may be employed (a) to reduce streaking from out-of-plane structures, which streaking may occur in a 2D reconstruction, and (b) to determine the out of plane structures. In an embodiment, the orientation of vessels or structures crossing through the imaging plane may be determined using the same technique followed by further analyzing for determining orientation of the vessels or structures.

As discussed above, in an embodiment, there is a simulation module capable of analyzing spatial representations of targets in a given volume (such as the estimated spatial representations produced by the reconstruction module) and generating acoustic signals that might be produced by applying electromagnetic energy to the given volume. In an embodiment, the simulation module produces at least two separate acoustic signals for a given volume: a simulated DAR signal that might be produced by temporal stress confinement of electromagnetically absorbent targets in the given volume (such as the electromagnetically absorbent targets discussed above); and a simulated SAR signal that might be produced by incidence of one or more acoustic wavefronts on acoustically reflective targets within the given volume (such as the acoustic wavefronts and acoustically reflective targets discussed above). In an embodiment, the DAR and SAR simulations are performed independently, such that the simulation module may simulate each component separately. In an embodiment, the electromagnetic energy directed to the volume is light energy and the simulated DAR signal produced by the simulation module is a simulation of the portion of the opto-acoustic response that would propagate through the volume essentially directly to the receivers. In an embodiment, the simulated SAR signal is a simulated ultrasound (US) backscatter signal produced by backscatter of an acoustic wavefront(s). In an embodiment, the acoustic wavefront(s) originates at or proximate to the surface of the volume and may cause ultrasound backscatter. Ultrasound backscatter can be modeled as a linear system and approximations to treat an unknown scatter field with a single or dual parameter model can be used. In an embodiment, different processes or parameters may be used to simulate the separate acoustic signals. In an embodiment, different and/or varying parameters may be used for the speed at which sound travels through the volume. In an embodiment, a value for the speed of sound in the volume is developed from previous testing, analysis, or computation. In an embodiment, a presumed, known, or computed speed of sound profile or propagation delay profile is provided as input to the simulation (and/or reconstruction) module(s).

In an embodiment, it can be assumed that the acoustic receiver and the origin of the acoustic wavefront are at substantially the same distance (r) from targets in the volume. Such an assumption represents a close approximation where the origin of the acoustic wavefront is quite proximal to a probe (e.g., a shallow skin layer, etc.) when compared to the depth of one or more of the targets. Where the electromagnetic energy is light energy, it may be assumed that the time required for the light energy to reach the targets in the volume and cause temporal stress confinement is negligible. Thus, it is inferred that sound energy in the DAR signal, which only travels from the targets, will reach the receiver after traversing the distance (r). While, sound energy in the SAR signal, which must first travel from the wavefront source to the targets and then from the targets to the receiver, will reach the receiver after traversing twice the distance (r + r). Based on these assumptions, about half the speed of sound (r/2r) is used to simulate the SAR signal to account for the increased distance the sound energy must travel through the volume.

In an embodiment, it can be assumed that the acoustic wavefront travels a depth (y) from its source to the targets in the volume, but an attempt is made to account for the fact that the acoustic receiver may be positioned at an angle (theta) to the depth vector (y) traveled by the acoustic wavefront. Thus, it is assumed that the sound energy in the DAR signal travels the distance (r), while the sound energy in the SAR signal travels the distance (r) in addition to the depth (y). Hence, the total distance traveled (y + r) can be calculated as r(1 + cos(theta)). In an embodiment, a slower speed of sound is used to simulate the SAR signal to account for the additional distance (y) traveled by the sound energy in that signal. In an embodiment, the speed of sound used to simulate the SAR signal is set at about 1/cos(theta) times the speed of sound. In an embodiment, a measured or presumed speed of sound profile is used to calculate the expected propagation times for one or more of the acoustic signals. In this configuration, the SAR may interfere with the DAR.

In some reflective mode or transmission mode configurations, it may be possible to position the energy source and receiver such that SAR due to scatter and DAR do not substantially interfere, but in other situations it is not possible. In an embodiment, an acoustic wavefront may be used to compute the speed of sound prior to or during component separation. In an embodiment, this wavefront may be produced proximate to the surface of the volume when the probe is configured in a reflective mode. In an embodiment, this wavefront may be produced as a result of the application of electromagnetic energy to passive elements on, in, or near the probe or the volume. In an embodiment, the probe includes ultrasound transducers (which may also act as the receiver discussed above) and the wavefront is produced by the transducers. Component separation itself may facilitate computing the speed of sound when reflective mode passive elements are used by separating interfering components of the acoustic signal. In an embodiment, the acoustic wavefront may originate from a handheld probe. In an embodiment, an array of receivers are used and the propagation times for reconstruction are adjusted separately based on the speed of sound profile and a measured or presumed propagation time to the receiver from the source of the sound. In an embodiment, the propagation times used are adjusted separately based on the speed of sound profile and a measured or presumed propagation time for each pixel or element in the spatial representation. In an embodiment, the propagation times used are adjusted separately based on the speed of sound profile and a measured or presumed angle for each angular ray of the spatial representation.

The following processing steps are an illustrative embodiment of an algorithm for simulating DAR, which can be adapted to simulate SAR (and/or PAB and/or ASW as further discussed below), using a look-up-table approach:

> a. Allocate a three dimensional array to store a look-up table where each value in the table corresponds to y-axis pixel depth coordinate in an image, and the table is indexed by sample number, x-axis pixel coordinate, and transducer channel.
> b. For each combination of sample number, x-axis pixel coordinate, and transducer channel, set the corresponding value in the table to the corresponding y-axis coordinate in the image. This can be determined by:
>
>> i. determining the expected distance travelled, which is the current sample number divided by sampling rate times speed of sound;
>> ii. determining the x-axis distance between the current x-axis pixel coordinate and the current transducer channel;
>> iii. determining the y-axis depth using the Pythagorean theorem which yields the result as the real part of the square root of the square of distance travelled less the x-axis distance; and
>> iv. converting the y-axis depth to a y-axis pixel coordinate and storing the result in the table.
>
> c. For each combination of sample number, x-axis pixel coordinate, and transducer channel, allocate a weight table and determine the weight for the table. If the y-axis depth is greater than zero and less than a maximum then the weight may correspond to the weight used by weighted delay-and-sum reconstruction (described below), otherwise a value of zero may be used for the weight.
> d. Allocate an output sinogram array and set all values to zero.
> e. Input an array corresponding to the spatial representation that is to be simulated.
> f. For each combination of sample number, x-axis pixel coordinate, and transducer channel:
>
>> i. determine the corresponding y-axis pixel coordinate from the lookup table;
>> ii. determine the corresponding weight value from the weight table by:

1. retrieving the value corresponding to the current x-axis pixel and looked-up y-axis pixel for the input spatial representation;
2. multiply the retrieved value by the corresponding weight value; and
3. adding the result of the multiplication to the sinogram element corresponding to the current transducer channel and sample number; and

g. If applicable, apply a shift invariant or shift variant filtering to the channels of the sinogram

In the above illustrative embodiment, steps a) through c) may only need to be computed one time. In an embodiment, the weights from step c) may be the same as the weights from weighted delay-and-sum reconstruction, or the backward projection, in which case, the simulation will approximate the adjoint operation of the reconstruction. In an embodiment, the SAR simulation may use a different speed of sound as a surface approximation, such as half the speed of sound. In an embodiment, the SAR simulation may replace step b.iii.) above for determining the depth in the y-axis with determining depth in the y-axis from the geometry as the square of distance travelled less the x-axis distance all divided by two times the distance travelled, which takes into account that the wavefront must travel from the surface to the acoustic target and then travel to a transducer. In an embodiment, the shift invariant or shift variant filtering can be used to model reflections from a coded wavefront, the filter coefficients may be determined in relation to an expected impulse response of the probe. In an embodiment, the coded wavefront may be based on a measured skin response, or other such coding from probe features as described below. In an embodiment, the filtering may be performed in step f.ii.3) and the adding of a filtered result may affect multiple sinogram elements. In an embodiment, the entire output sinogram may be shifted by a number of samples to compensate for a delay with respect to the timing of an energy event. In an embodiment, the look-up-table and weights calculation is replaced by a fast optimized computation computed on the fly. In an embodiment, the filtering may apply a spatially dependent impulse response applicable to SAR.

As discussed above, in an embodiment, the processing subsystem includes a reconstruction module capable of analyzing acoustic signals received from a volume of tissue (such as the DAR signal and the SAR signal discussed above) and producing spatial representations of the volume. In an embodiment, the reconstruction module estimates positions of targets as spatially represented in the volume (such as the electromagnetically absorbent targets and the acoustically reflective targets discussed above). In an embodiment, the acoustic signals are provided in the form of one or more sinograms containing processed or unprocessed acoustic data. In an embodiment, the reconstruction module is capable of producing a least two separate spatial representations of a volume from a given acoustic signal or sinogram. In an embodiment, the reconstruction module can be applied to produce both a DAR and a SAR representation of the volume from a given sinogram. Various reconstruction methods are known in the art. Exemplary reconstruction techniques are described below.

Figure 8A is a block diagram illustrating the process flow associated with a reconstruction module in accordance with an embodiment. Although the term "reconstruction" as used herein refers to a process or module for converting the processed or unprocessed data in a sinogram into an image (or other spatial representation) representing localized features in a volume, it is important to understand that such reconstruction can be done at many different levels. For example, reconstruction can refer to a simple function that converts a sinogram into an image representation such as through the use of the weighted delay-and-sum approach described next. Or, in an embodiment, reconstruction can refer to a more complex process whereby a resultant image representation is improved by applying a reconstruction function or module at a different level of abstraction (also referred to here as "auxiliary reconstruction") along with any other signal or image processing techniques. Consequently, a reconstruction algorithm may include an auxiliary reconstruction processing stage, as shown in Figure 8A.

As an example, an iterative reconstruction algorithm may apply an auxiliary reconstruction function two or more times. In an embodiment, component separation can itself be part of a larger reconstruction function because part of improving a reconstructed image of the volume may include separating (e.g., removing) unwanted components of the sinogram. Various applications of reconstruction with component separation are shown in Figures 9A through 9D. In each of these figures, the process encompassed by the dotted line can itself be considered a "reconstruction" as the input is a sinogram and the output is an image. Although, in the examples illustrated in Figures 9A through 9D, each process produces two separate images (as further described below). In an embodiment, one of the two separate images may be ignored, discarded or used for other purposes. In the embodiment of Figure 9A, a component separation process receives sinogram data as input and outputs a DAR image and a SAR image. In the embodiment of Figure 9B, a process includes an auxiliary reconstruction process and a component separation process. The auxiliary reconstruction process receives as input the sinogram data and produces as output a combined image. A component separation process then receives the combined image as input and outputs a DAR image and a SAR image. In the embodiment of Figure 9C, a process includes an auxiliary reconstruction process, an initialize values process and a component separation process. The auxiliary process takes as input the sinogram data and outputs a DAR image. The initialize values process outputs a SAR image. A component separation process receives as input the DAR image and the SAR image, and outputs a DAR image and a SAR image. In the embodiment of Figure 9D, a process includes a component separation process, a first auxiliary reconstruction process, and a second auxiliary reconstruction process. The component separation process receives as input the sinogram data and outputs a DAR sinogram and a SAR sinogram. The first auxiliary reconstruction process receives as input the DAR sinogram and outputs a DAR image, while the second auxiliary reconstruction process receives as input a SAR sinogram and outputs a SAR image.

In an embodiment, reconstruction can be based on a weighted delay-and-sum approach. In an embodiment, the weighted delay-and-sum approach implements a backward projection. The weighted delay-and-sum algorithm may optionally be preceded by a transform operator. In an embodiment, the weighted delay-and-sum algorithm can operate on complex-valued data. In an embodiment, weights may be used by reconstruction to represent the contributions from each sample to be used for each pixel, and organizationally, the method used to generate the weights may be considered part of image reconstruction. In an embodiment, the weights may be tuned based on an analysis of the collected data.

Generally, reconstruction takes as input processed or unprocessed channel data, i.e., a sinogram, and uses this information to produce a two dimensional image of a predetermined resolution.

The dimensions of an individual pixel (in units of length) determine the image resolution. If the maximum frequency content in the sinogram data is too high for the selected resolution, aliasing can occur during reconstruction. Thus, in an embodiment, the resolution and sampling rate may be used to compute limits for the maximum frequency content that will be used in reconstruction, and thus to avoid frequency content that is too high for the selected resolution. In an embodiment, the sinogram can be low-pass filtered to an appropriate cutoff frequency to prevent or mitigate aliasing.

Conversely, if the sampling rate is too low to support the image resolution, then, in an embodiment, the sinogram can be upsampled and interpolated so to produce a higher quality images. While the two dimensional image can be any resolution, in an exemplary embodiment, the image can comprise 512x512 pixels. In an embodiment, the image can comprise 1280x720 pixels. In yet another exemplary embodiment, the image may comprise 1920x1200 pixels. In an embodiment, the horizontal resolution is at least 512 pixels wide, and may be up to 2560 pixels wide or more, and the vertical resolution is at least 512 pixels high, and may be up to 1600 pixels high or more. In an embodiment, the image resolution conforms to the resolution of an existing display device or standard, or a known storage format, e.g., 640x480, 800x600, 1280x1024, 1280x720, 1920x1080, 1920x1200, 2560x1600, 3840x2160, 4096x2160, 4096x1714, 3996x2160, 3656x2664 and/or 4096x3112. Generally, a processing time (and thus performance) and/or memory constraint tradeoff is required to attain higher resolution.

A two dimensional image may represent variations in the volume, such as structures, blood, or other inhomogeneities in tissue. The reconstruction may be based upon the first propagation time from each location in the tissue to each transducer and the contribution strength of each sample to each pixel. The signal intensities contributing to each pixel in the image are combined to generate the reconstruction.

In an embodiment, the DAR and SAR reconstructions are performed independently, such that the reconstruction module may simulate each component separately. The following processing steps are an illustrative embodiment of a reconstruction algorithm using a weighted delay-and-sum technique for DAR (that can be adapted to reconstruct SAR and/or ASW):

h. Allocate an output image array and set all values to zero;
i. For each transducer channel:

i. For each pixel in the output image array:

1. Access the delay (in samples) from Sample Delay Table for that channel and pixel, and then retrieve the sample (from the sinogram) corresponding to the channel and delay;
2. Access the weight from Weights Table corresponding to the channel and pixel;
3. Multiply the sample by the corresponding weight; and
4. Add and store the result with in location of the output image array corresponding to the destination pixel.

The weights table is a table representing the relative contribution of each sample in the sinogram to each pixel in the resulting image. In an exemplary embodiment, for relative computational efficiency, the same weights table can be used for the real and imaginary components of a complex sinogram. In an embodiment, separate weights table can be used for each of the components of a complex sinogram. In an embodiment, one complex weights table can be used for the real and imaginary components of a complex sinogram. In an embodiment, separate complex weights table can be used for each of the components of a complex sinogram. In an embodiment, a complex weights table can be used to account for standing-wave type patterns in the image that are the result of the system geometry.

The weights table can be used to establish something akin to an aperture in software. Thus, in an embodiment, where a wider aperture is desired, more weight is given to off-center samples. Stated in other words, for example, for a given transducer, usually no sample would be given more weight than the sample directly beneath the transducer, and for the purposes of illustration, consider that the weight for a given sample directly beneath the transducer is 1. Consider further the relative contribution of samples that are at 15, 30 and 45 degrees from center, but equidistant from the transducer. To narrow the aperture, those samples could be weighted 0.5, 0.25 and 0.12 respectively, while to widen the aperture, those same samples could be weighted 0.9, 0.8 and 0.7 respectively. The former would provide only a slight (12%) weight to samples received from a source at 45 degrees from center, while the latter would provide the same sample much higher (70%) weighting. In an embodiment, the system displaying the opto-acoustic output- which may, but need not be the same as the system acquiring the sinogram- would provide the operator the ability to vary this parameter (i.e., the software aperture) when viewing opto-acoustic images.

In an embodiment, a very large table contains a mapping of relative weight and delay for each pixel and transducer. Thus, in an embodiment where a target image is 512x512 pixels and the probe 102 has 128 channels (i.e., transducers), there are 33,554,432 weight entries and the same number of delay entries. Similarly, in an embodiment where a target image is 1280x720 pixels and the probe 102 has 128 channels (i.e., transducers), there are 117,964,800 of each type of entry. In an embodiment where a target image is 1920x1200, and the probe has 256 channels, there are almost 600 million of each type of entry. Thus, as mentioned above, a processing time (and thus performance) and/or memory constraint tradeoff is generally required to create a target image having a higher resolution.

Image Reconstruction - Calculate Weights and Delays

As discussed above, in the illustrative embodiment of a delay-and-sum reconstruction algorithm, a Weights Table may be employed. An algorithm may be used to calculate the Sample Delay Table and Weights Table for each transducer. In an embodiment, the data comprising Sample Delay Table(s) correlates the estimated contribution of each transducer to each pixel, while the data comprising the Weight Table(s) provides an estimate of the relative weighting of the contribution of each transducer to each pixel as compared to the other contributions to that pixel. In an embodiment, the Weights Table may be used to account for angular apodization with respect to the transducer's norm, power of the laser, time gain control, light attenuation within the tissue, skin thickness, coupling medium characteristics, patient specific variables, wavelength specific variables and other factors.

In an embodiment, each of the tables corresponds in size (in pixels) to the two dimensional image output by image reconstruction, and a plurality of each table are created, one for each channel. In the illustrative embodiment above, each Sample Delay Table correlates the pixels of the target image with the samples in an sinogram, thus, one Sample Delay Table (which is specific to a channel) will identify for each pixel in the image, the specific sample number in that channel that is to be used in calculating that pixel. Similarly, in the illustrative embodiment above, each Weights Table correlates the pixels of the target image with the weight given to the sample that will be used; thus, one Weights Table (which is specific to a channel) will identify for each pixel in the image, the weight to be given to the sample from that channel when calculating the pixel.

X- and Y- coordinates of the image pixels are calculated using the input information on the image size and location. In an embodiment, the time delays for DAR are calculated for each transducer and each pixel by knowing the distance between pixel and transducer and the speed of sound. If an acoustic matching layer with different speed of sound is used, then separate time delays are calculated inside and outside of the matching layer and added together, resulting in the overall transducer-pixel delay. The weights are calculated for each transducer and each pixel, depending on their relative location. The distance and angle between the transducer-pixel vector and transducer's norm are taken into account, as well as the depth position of an individual pixel. In an embodiment, the system calculating the weights and/or delays- which may, but need not be the same as the system acquiring the sinogram or displaying the images reconstructed there-from- would provide the operator the ability to vary parameters used in processing. In an embodiment, the system calculating the weights would provide the operator the ability to vary the bases for the weight calculation, thus, e.g., giving more or less weight to off-center acoustic data. In an embodiment, the system calculating the weights would provide the operator the ability to controls whether linear or power relationships are be used in calculation of the weights.

In an embodiment, the SAR component may have a separate weights table, or a separate delays table from DAR. In an embodiment, the SAR delays table may be computed such that the time delays reflect the distance of an acoustic wave that travels from the surface to the target and then to a transducer. Thus, the time delays are calculated for each transducer and each pixel based on the distance between the pixel and the transducer, the speed of sound (or an estimate thereof), and the depth of the pixel. In an embodiment, the weights table for SAR may account for the acoustic attenuation of the wavefront as it propagates to the depth of the pixel. In an embodiment, the weights for a pixel to a transducer for DAR may be computed as the depth of the pixel divided by the distance from the pixel to the transducer all raised to a cubed power and multiplied by an exponentially decaying function of the pixel depth. In an embodiment, the weights for a pixel to a transducer for SAR may be computed as the depth of the pixel plus the distance from the pixel to the transducer all divided by the distance from the pixel to the transducer all raised to a cubed power multiplied by an exponentially decaying function of the pixel depth plus the distance from the pixel to the transducer.

Once reconstruction is complete, post-processing may be performed on the resulting image or images.

In an embodiment, image reconstruction may be based on Adaptive Beamforming, Generalized Sideband Cancellation, or other methods as are known in the art. In an embodiment, techniques for reconstruction may be based on determining cross-correlations functions between channels and/or maximizing a sharpness objective of the image.

In an embodiment, a method to reconstruct a volume may consist of decomposing a cross-section or volume into radial wavelets, the radial wavelets representing opto-acoustic sources (the measured opto-acoustic return signal of radial opto-acoustic sources in particular are presumed to obey a simple closed form equation), the technique of Wavelet-Vaguelette decomposition may be used to relate the wavelets and vaguelettes between the image domain and the sinogram and to thereby determine the intensities of the radial wavelets in the image, and thus to reconstruct the image. In an embodiment, the projection of radial wavelets from the image domain into the sinogram domain (i.e., vaguelettes) can be used in conjunction with other image formation techniques prior to determining the intensities of the radial wavelets. In an embodiment, adaptive beamforming, or wavelet de-noising involving thresholding can be performed on the radial-wavelet projections as a stage of such a reconstruction.

Iterative reconstruction involves applying a reconstruction (and/or simulation) operation(s) one or more times to move closer to a solution. In an embodiment, reconstruction may be based on Iterative Minimization or Iterative Maximization, such as, for example, L1-minimization or L2-minimization. Iterative Minimization algorithms for reconstruction and enhancement require high computational load and thus, are often not considered applicable for real-time imaging. Nevertheless, in accordance with embodiments disclosed herein, in some circumstances, it is feasible for real-time opto-acoustic reconstruction of a cross-section of a volume to be performed using an L1-minimization algorithm. In an exemplary embodiment for performing L1-minimization reconstruction in real-time on a 2D cross-section of a volume, the Fast Wavelet Iterative Thresholding Algorithm is used, and combined with the Helmholtz wave equation in the frequency-domain, which can be efficiently used to represent opto-acoustic wave propagation yielding a diagonalizable (or nearly diagonalizable) system matrix. In an embodiment, the pixels of the image may be decomposed into radial wavelets, the decomposition represented in the frequency domain as radial subbands, and the radial subbands used in the iterative thresholding. See, e.g., WO2013056089. In an embodiment, each sub-band of the representation may be reconstructed and/or simulated substantially independently. In an embodiment, the iterations may be performed on sub-bands independently as though each sub-band is a separate iterative reconstruction problem. In an embodiment, a Fast Wavelet Iterative Thresholding Algorithm or Fast Weighted Iterative Soft Thresholding Algorithm may be used where the system matrix is found empirically rather than through using an ideal equation.

When the laser illuminates the volume of tissue with at least a portion of the surface being adjacent to a medium that is not perfectly matched to the acoustic properties of the volume, the propagating acoustic wave may reflect - at least in part - off the unmatched surface and propagate into the volume as an incident wave-front. The incident wave-front can further reflect off acoustic discontinuities in the tissue and interfere with the opto-acoustic return signal creating an artifact. This artifact can be separated from the opto-acoustic return signal using, e.g., an iterative minimization technique. In an embodiment, an image mapping the intensity of this artifact can be produced. In an embodiment, the image mapping the intensity of this artifact is an image of a SAR component.

In an embodiment, a pattern detection classifier can be applied to an opto-acoustic return signal, wherein the classifier output reflects the strength of a particular indicator as a function of time (or distance). Accordingly, upon obtaining measurements from multiple transducer positions, the classifier output can be beam-formed to localize the source (i.e., phenomenon) causing the pattern detected. An image produced from the beam-formed classifier output may suffer from blurring, reconstruction artifacts, and streak artifacts, which may be particularly acute in a limited-view case. These artifacts may result at least in part because the pattern classified signal may lack information concerning signal strength that is part of a non-pattern classified sinogram, and its intensity is related to the presence of the pattern, not necessarily on the distance that the transducer is located from the source of the pattern. The classifier output of a classified opto-acoustic signal, however, can be "fit" into the propagation model of the Helmholtz equation where the classifier output is characterized as originating from an instantaneous source term at a given position. Thus, to reduce the streaking, blurring and artifacts a parametric map of the pattern classified signal can be formed using techniques for reconstruction and deconvolution other than simple beamforming. Application of, e.g., an iterative minimization technique can be used to reduce streaking and thus better localize the source of the pattern. Different types of classifiers and reconstruction techniques may have different considerations that apply. In an exemplary embodiment, a parametric map of the classified quantity can be produced by using an iterative minimization technique, where the system matrix is formed as it would be had the source been an opto-acoustic signal. In an embodiment, the sparse basis representation used by, e.g., L1 minimization, may serve to localize the source of the pattern and hence reduce artifacts. Thus, rather than applying the reconstruction technique to an opto-acoustic return signal, it may be applied to classifier output, where the classifier output is represented in the form of a sinogram. In an embodiment, the reconstruction technique is applied as though the classifier output were an opto-acoustic return signal. In an embodiment, further processing, such as taking a complex envelope of the classifier output, filtering, or deconvolving the classifier output may be performed prior to reconstruction. In an embodiment, the classifier may be designed to discriminate between normal and abnormal branching blood vessels in tissue. In an embodiment, the pattern detection classifier may be used to detect signals resulting from a coded probe as described below.

In an embodiment, the reconstruction module is capable of producing a least two separate spatial representations of a volume from a given acoustic signal. In an embodiment, the reconstruction module returns a first spatial representation based on the assumption that the given acoustic signal was produced by temporal stress confinement of electromagnetically absorbent targets in the volume (such as the electromagnetically absorbent targets discussed above) and returns a second spatial representation based on the assumption that the given acoustic signal was produced by scatter of one or more acoustic wavefronts off acoustically reflective targets within the volume (such as the acoustic wavefronts and acoustically reflective targets discussed above). Thus, the given acoustic signal can be a DAR signal or a SAR signal. A given acoustic signal may contain both DAR and SAR components and thus, the reconstruction module can be applied to generate a reconstructed DAR spatial representation and a reconstructed SAR spatial representation for the given acoustic signal. See, for example, a) and e) of Figures 10 and 11. Where the electromagnetic energy is light energy, the DAR signal includes portions of an opto-acoustic signal produced by temporal stress confinement, while the SAR signal can include an ultrasound backscatter signal produced by backscatter of an acoustic wavefront. In other words, where a given acoustic signal has both opto-acoustic and ultrasound components, the reconstruction module can be applied to generate a reconstructed opto-acoustic spatial representation and a reconstructed ultrasound spatial representation for the given acoustic signal. The techniques, calculations, inferences, and assumptions discussed above with respect to simulation can also be applied to reconstruction. In an embodiment, a weighted delay-and-sum technique may be applied to reconstruct the DAR and/or the SAR signals. Figure 10 shows a series of images illustrating an example of SAR/DAR component separation applied to a digital phantom with a DAR and SAR target. Figure 11 shows a series of images illustrating an example of SAR/DAR component separation applied to data from a breast lesion.

Simulation and Reconstruction of Acoustic Return and Probe Acoustic Backscatter

When comparing the simulation and reconstruction between DAR and SAR, it can be noted that in embodiments the wavefront may propagate from a probe interface or from the surface of the volume directly beneath or outside the probe and travel down through the tissue to reach the acoustic target that will backscatter creating probe acoustic backscatter (PAB). In the case of a theoretically ideal simple incident wavefront directed downwards into the tissue, the incident wavefront will reach a position in the tissue in direct proportion to the depth of the position based on the speed of sound. Call this position (x,y). A transducer element, located on the probe or elsewhere, may be distance r away from (x,y). The PAB from the position with reach the element after propagating distance y+r. The acoustic return from (x,y) will reach the element after only propagating distance r. In an embodiment, the SAR is substantially assumed to consist of PAB. Generally, SAR contains signals in addition to PAB.

In a delay and sum reconstruction algorithm, in an embodiment, the delays for DAR will be based on r. The delays for PAB, in an embodiment, will be based on y+r. In an embodiment, this is calculated in terms the angle theta between the surface normal and the probe element through the position. The PAB is then y+r = r(1+cos(theta)). In an embodiment, the delay can be approximated by assuming that the distance for PAB is twice the distance of the DAR. This simplification holds for small theta, and has some further applicability due to angular dependence. In an embodiment, the same reconstruction can be used for PAB and DAR, but with different speeds of sound to account for the differences in delay.

In an embodiment, the processing subsystem comprises a point spread function (PSF) module capable of applying a model of the system to spatial representations. In an embodiment, a PSF module applies the simulation and reconstruction modules discussed above to process given first and second spatial representations of targets in a volume. In an embodiment, the first and second spatial representations are DAR and SAR spatial representations respectively. In an embodiment, the PSF module first applies the simulation module: to the first spatial representation to produce a DAR signal that might be produced by the first spatial representation; and to the second spatial representation to produce a SAR signal that might be produced by the second spatial representation.

Next, the PSF module combines the DAR and SAR signals to produce a combined acoustic signal. In an embodiment, the DAR and SAR signals may be added to produce the combined signal. In an embodiment, the DAR and SAR signals may be processed before they are combined, and/or the combined acoustic signal may be processed after the combination. Various methods for such processing including weighting and thresholding are discussed below.

Subsequently, the reconstruction module may be applied to the combined acoustic signal to produce a PSF spatial representation of the DAR component and a separate PSF representation of the SAR component. See, for example, d) and h) of Figures 10 and 11. In an embodiment, the first and second spatial representations are opto-acoustic and ultrasound spatial representations, respectively. A mixing matrix can be used to describe combinations of DAR and SAR signals. In an embodiment, multiple sinograms may be collected (e.g. for multiple wavelength data), and the PSF module can use a mixing matrix to linearly combine the DAR and SAR signals. Block-level process flow charts for three alternative embodiments of aspects of the PSF module are shown in Figures 12A through 12C. Figure 7A shows an exemplary DAR/SAR PSF embodiment. Figure 12B shows an alternate DAR/SAR PSF embodiment. Figure 12C shows an embodiment of a pathway for additional processing. In the embodiment of Figure 12A, the DAR image is simulated with the DAR simulation module to produce a DAR sinogram, and the SAR image is simulated with the SAR simulation module to produce a SAR sinogram. The DAR sinogram is combined with the SAR sinogram to produce a combined sinogram. The combined sinogram is then reconstructed using a DAR reconstruction to reconstruct a DAR portion of the PSF ouput and using a SAR reconstruction to reconstruct a SAR portion of the PSF output. In the embodiment of Figure 12B, an alternate expanded version of a PSF module is shown. In this case, separate DAR and SAR reconstructions are performed on each of the SAR and DAR sinograms and the reconstructed SAR/DAR, SAR/SAR, DAR/DAR, and DAR/SAR parts are combined in a manner to produce an appropriate PSF output representation. The embodiment of Figure 12C is another alternate embodiment of performing PSF processing. In this case, SAR/DAR, SAR/SAR, DAR/DAR, and DAR/SAR parts are simulated to produce sinograms. Processing of each sinogram may occur and the output of the processing may include further processing and/or combining of the processed sinograms. The outputs from the combining and/or processing are reconstruced using a DAR reconstruction path and a SAR reconstruction path. The outputs correspond to SAR/DAR, SAR/SAR, DAR/DAR, and DAR/SAR parts. When SAR/DAR is merged with DAR/DAR and DAR/SAR is merged with SAR/SAR, Figure 12C will resemble Figure 12A. Figure 12C indicates that each PSF output depends on at least one PSF input. In an embodiment, each PSF output is implemented by calling an optimized processing block to operate on the relevant PSF inputs.

In an embodiment, the processing subsystem comprises an error calculation module capable of measuring residual error between two sets of data in the spatial representation domain, two sets of data in the acoustic signal domain, and/or between two sets of data across mixed domains. In an embodiment, measuring residual error occurs between transformed domains. In an embodiment, a processed spatial representation is subtracted from a reference spatial representation to produce a residual error between the two representations. In an embodiment, the input to, or output of, the error calculation module may be weighted or thresholded as further discussed below. In an embodiment, error calculation may be performed in the signal domain. When error calculation is performed in the signal domain, a reference may be represented in the signal domain rather than as a spatial representation. In an embodiment, the error calculation may be performed in the signal domain from within the point spread function module after spatial representations are converted to the signal domain. In the signal domain it is easier to account for time delay offset between the current estimate and the measured data; thus, accounting for propagation time delay offset of each channel, or performing aberration correction, may be more efficient and/or more accurate in the signal domain.

In an embodiment, the processing subsystem comprises a correction module capable of adjusting a spatial representation of a given volume based on given residual error. In an embodiment, a separate residual is provided for each pixel in the spatial representation and the residuals are simply added to each pixel in the spatial representation. In an alternate embodiment, a single residual is provided for the entire spatial representation. In other embodiments, a plurality of residuals is provided and the spatial representation is adjusted by wavelets, sub-bands, or other channels. In an embodiment, the given residuals are weighted before they are added to the given spatial representation. Various methods for weighting are known in the art. In an embodiment a single constant weight is used across the entire image. In an embodiment, weights are varied based on a weights table as discussed above. In an embodiment, weights are varied by channel or sub-band. Weights can also be varied by wavelet as will be apparent to one skilled in the art. In an embodiment, weights are chosen that exceed a value required to obtain convergence on iteration, as further discussed below. Such weights may be determined by experimentation.

In an embodiment, the processing subsystem also comprises a component separation module capable of applying the simulation, reconstruction, point spread function, error calculation, and/or correction modules discussed above to separate at least two components of a given acoustic signal. In an exemplary embodiment, the given acoustic signal is separated into DAR and SAR components. In an embodiment, the given acoustic signal is separated into OA and US components.

In an embodiment, the reconstruction module is applied to the given acoustic signal to produce a reference DAR spatial representation and a reference SAR spatial representation of a volume that produced the given acoustic signal. The reference spatial representations can also be used as initial values for an initial DAR spatial representation and an initial SAR spatial representation respectively. In another embodiment, the DAR and SAR spatial representations can be initialized to all zeros, threshold values, weight values as discussed above, or other specified values. The point spread function module can then be applied to the initialized DAR and SAR spatial representations to produce PSF DAR and PSF SAR spatial representations of the volume. The error calculation module can be applied to determine the residual error between the reference and the PSF DAR spatial representations. The error calculation module can be similarly applied to determine the residual error between the reference and the PSF SAR spatial representations. The correction module can then be applied to correct the initial DAR and initial SAR spatial representations based on the residuals to produce refined DAR and refined SAR spatial representations of the volume.

The component separation module can be applied to produce separate images of electromagnetically absorbent and acoustically reflective targets in the volume (such as the electromagnetically absorbent and acoustically reflective targets discussed above). See, for example, b) and f) of Figures 10 and 11. Better results may be obtained when thresholding is applied. See, for example, c) and g) of Figures 10 and 11. In another aspect of the disclosure, the above steps are applied to a given acoustic signal as a process with or without the provided system.

In an embodiment, the new spatial representations are further refined by iteratively applying the component separation module one or more additional times. In an embodiment, the refined DAR and refined SAR spatial representations become the initial DAR and initial SAR spatial representations for the next iteration of the process. The component separation may be iteratively applied until some condition is met. In an embodiment, the component separation module is iteratively applied a predetermined number of times. In an embodiment, the component separation module is iteratively applied until the measured residuals reach a specified limit. In an embodiment, the component separation module is iteratively applied until the PSF spatial representations converge with the reference spatial representations. In an embodiment, the effects of one or more divergent elements of the acoustic signals are removed as the modules are iteratively applied. Various methods for recognizing convergence and removing divergent effects can be used to carry out aspects of the subject invention, and will be apparent to one of skill in the art in the context presented herein. Examples of both hard and soft thresholding may be found in A Fast Wavelet-Based Reconstruction Method for Magnetic Resonance Imaging, by Guerquin-Kern, et. al, IEEE Transactions on Medical Imaging, Vol. 30, No. 9, September 2011, at 1649.

In an embodiment, thresholding (which may be hard or soft thresholding) is applied based on the weight values discussed above and in proportion to a regularization parameter. In an embodiment, pixel values below a specified threshold are zeroed, while other values can be reduced in magnitude. In an embodiment, weights can be applied to the entire image, sub-bands, wavelets, or channels as discussed above. In an embodiment, the thresholding operation is a denoising operation, as wavelet denoising can be similar or the same as thresholding. Various denoising techniques can be used with the subject invention including, but not limited to those described in WO2013056089.

In an embodiment, simulation may be implemented by applying a system transfer matrix. A simple backprojection reconstruction may be represented as the Hermitian adjoint (i.e. conjugate transpose) of the system transfer matrix. Thus, when the Hermitian adjoint of the system transfer matrix is applied to measurement data from detectors (or signals in this domain) to reconstruct a volume, the result can be considered a reconstruction that maps the data domain to the solution domain. Iterative minimization may produce a result of higher quality than using a pseudo-inverse or other reconstruction method. Iterative minimization can be performed by computing a residual (e.g., difference) between a reference and a relationship of a current estimate applied to the system to modify the current estimate of the system. In this sense, the current estimate may move closer and closer towards an actual solution.

For the case of a multi-parameter model, a system transfer matrix may be formed with a block matrix approach by forming a matrix out of sub-matrices. If the model is dependent on each parameter independently, then separate system transfer matrix models may be separated out and computed independently under superposition.

The independent separation described above may not be optimal in solving the concentration of a chromophore in a multi-wavelength opto-acoustic system. In a multi-wavelength opto-acoustic system, the presence of the chromophores affects each channel (due to the wavelength specific absorption of the chromophore), and thus, the channels are not independent. In this example, the system transfer matrix is not considered (to the same degree) a reconstruction process. Often, in a reconstruction process, the goal is to use boundary measurements from a detector to literally reconstruct a spatial representation of the volume from the measurement data. If each pixel in an image is treated on substantially the same footing when a point spread function is applied, the point spread function can be considered spatially invariant (e.g. the point spread is the same for every position). This can yield a simplified model. However, the spatially variant effects (e.g. image streaking that can occur as a result of the imaging device or its measurement geometry in a reconstruction process) may be important. In exemplary circumstances, the separation of DAR from SAR (or other such components) is facilitated by the presence of these spatially variant effects, which may manifest differently for each component in an image since each component can have a different reconstruction process.

Techniques for finding concentrations of known or unknown chromophores will be apparent to one skilled in the art. In an embodiment, a Multispectral Morphological Component Analysis (MMCA) technique may be used, such as the one discussed in Bobin, et al. in Morphological Diversity and Sparsity for Multichannel Data Restoration, Journal of Mathematical Imaging and Vision, Vol. 33, Issue 2, pp. 149-168 (February 2009). For example, the problem can be treated as a spatially invariant image processing problem in the image domain. In this technique, one set of dictionaries represents the spectral aspect (each wavelength corresponds to a spectral observation) and another set of dictionaries represents the image aspect. In this problem, an image mixing problem as applied to hyper-spectral data can help to separate the components. Using this technique, chromophore component separation can be accomplished without modeling a reconstruction process. In the image domain, wavelets or dictionary elements that are spatially shifted copies of each other may be used for efficiency. In an embodiment, a multispectral Morphological Component Analysis (MCA) dictionary approach may also be used where dictionary symbols are projections on to a reconstruction operator. Such a multispectral MCA dictionary approach may be applied to chromophore component separation, since it is applicable to system transfer matrices. In this case, in an embodiment, separate DAR and SAR simulation, and reconstruction, could be used for efficient implementation.

Additionally, Morphological Component Analysis provides techniques for quantifying the performance of how well signals represented in different dictionaries may be separated based on the similarities between the dictionaries used.

These techniques can be applied to DAR and SAR components, and may be used to quantify how well a DAR signal may be separated from a given SAR signal by looking at the similarities of their PSF functions in a given component separation technique. More generally, the technique can be applied to the novel component separation methods disclosed herein to see how well one set of components can be separated from another. In an embodiment, component separation does not solely rely on accurately modelling the resulting DAR and SAR signals from targets during simulation. For example, in an embodiment, differences in signal arrival times from the targets are used to separate signal components. In an embodiment, the component separation process also takes into account how these differences in signal arrival times influence the respective dictionaries.

Independence of Acoustic Return and an Incident Wavefront

Returning to the discussion about separating the system transfer matrix. In an embodiment, the produced incident wavefront is presumed to be responsible for all acoustic backscatter (an approximation) and the other secondary acoustic scatter (a.k.a. other acoustic scatter, acoustic reflections) that reflect from the acoustic-return sources are ignored- and as a result, the system transfer matrix from the DAR can be treated independently from the reflected acoustic backscatter (SAR). In such embodiment, separate simulation and reconstruction can be performed on the reflected acoustic backscatter from the wavefront. In an embodiment, separate simulation and reconstruction of DAR and SAR signals yields faster simulations and reconstructions, since faster algorithms may be used for simulating each of these separately.

Exemplary Pseudo Code

Pseudo code follows that can be used to implement an aspect of an embodiment of the processing subsystem.

```
vn1 = a1 = reconstruct_DAR(recorded data from transducers);

vn2 = a2 = reconstruct_SAR(recorded data from transducers);

for n = 1:NUMBER_OF_ITERATIONS

        [vn1_psf, vn2_psf] = PSF(vn1, vn2);
        r1 = a1-vn1_psf;
        r2 = a2-vn2_psf;
        tmp1 = vn1 + tau1.*r1;
        tmp2 = vn2 + tau2.*r2;
        wnlB = threshold(tmp1, lambda, tau1);
        wn2B = threshold(tmp2, lambda, tau2);
        tnB = (1+sqrt(1+4*tn^2))/2;
        vnlB = wn1B+(tn-1)./tnB*(wn1B-wn1);
        vn2B = wn2B+(tn-1)./tnB*(wn2B-wn2);
        wn1 = wn1B;
        vn1 = vn1B;
        wn2 = wn2B;
        vn2 = vn2B;
        tn = tnB;

    end

    function [x1_psf x2_psf] = PSF(x1,x2)

        sinogram_tmp = simulate(x1, x2);
        [x1_psf, x2_psf] = reconstruct(sinogram_tmp);

    end

    function sinogram_combined = simulate(x1, x2)
    sinogram_combined = simulate_DAR(x1) + simulate_SAR(x2);
    end
```

```
function [x1_out, x2_out] = reconstruct(sinogram_tmp)

    x1_out = reconstruct_DAR(sinogram_tmp);
    x2_out = reconstruct_SAR(sinogram_tmp);

end
```

In this example, a1 and a2 are arrays (e.g., two or more dimensional arrays) holding DAR and SAR images reconstructed from the recorded acoustic signal. In the above embodiment, a1 and a2 are used as the reference images. The variables vn1 and vn2 are arrays for holding the current reconstructed DAR and SAR spatial representations respectively. The variables r1 and r2 hold pixel by pixel arrays of residuals. In other embodiments, a single residual can be calculated for the entire image or residuals can be calculated by wavelets, sub-bands, or other channels as discussed above. Here, the variables tau1 and tau2 are pixel by pixel weights that are applied to the residuals. In other embodiments, weights can be applied by wavelets, sub-bands, or other channels as discussed above. In an embodiment, the weights applied are based on the weights table discussed above. In the pseudo-code embodiment, thresholding is applied to the current DAR and SAR images based on tau1 and tau2 in proportion to the regularization parameter (lambda). In an embodiment, the a1 and a2 reference images are produced using a more complex reconstruction algorithm than that performed by the PSF function during iteration. This embodiment, allows the reference images to start off with a higher quality, while maintaining speed for the subsequent iterative processing. For example, in an embodiment, adaptive beamforming is used to reconstruct the a1 and a2 reference images. Figure 8 shows a process flow in an illustrative embodiment for SAR/DAR component separation.

In accordance with the embodiment of Figure 13, electromagnetic energy is first delivered to the tissue or other area of interest. A multiple-component acoustic signal is then received as all active detector positions. Then, a reference representation is constructed for each component of the signal. A current representation is then initialized for each component of the signal. An iterative PSF process is then applied as follows. A PSF function is applied to each current representation to create a PSF representation. Residual error is calculated from reference representations and the PSF representation. Current representations are then corrected based on calculated residuals. Thresholding is then applied, and the iterative process returns to the step of applying a point spread function above. After the iterative PSF process, the representations are output and/or stored.

Iteration, Weighting, Thresholding

Various iterative thresholding techniques are known in the art and can be applied to the subject invention including, but not limited to, hard thresholding, soft thresholding, FISTA (Fast Iterative Soft Thresholding), FWISTA (Fast Weighted Iterative Soft Thresholding), Morphological Component Analysis (MCA), Multispectral Morphological Component Analysis (MMCA). In an embodiment, values below a threshold are zeroed while other values remain the same or are reduced in magnitude. The weighting step can be optional. Alternately, if each pixel is not individually weighted, a constant value that corresponds to the maximum divergent value of tau1 and tau2 can be used. As described herein, and known in the art, sparse representation in transform domains or sparse dictionaries can be used to improve performance. Accordingly, some illustrative embodiments for using sparse representations in component separation are shown in Figures 14A through 14D. Figures 14A through 14D illustrate embodiments for applying dictionary transformations in component separation.

In accordance with the embodiment of Figure 14A, a reference representation is first constructed for each component of a signal for each frame. Then, a current representation is initialized for each component of the signal for each frame. An iterative PSF process is then applied as follows. A PSF function is applied to each current representation to create a PSF representation. Residual error is calculated from reference representations and the PSF representation. Current representations are then corrected based on calculated residuals. Thresholding is then applied, and the iterative process returns to the step of applying a point spread function above.

In accordance with the embodiment of Figure 14B, a reference representation is first constructed for each component of a signal for each frame. Then, a current representation is initialized for each component of the signal for each frame. A dictionary transformation is then applied to each current representation and/or reference representation. Then, an iterative process begins by applying a point spread function to each current representation to create a PSF representation. In an embodiment, this involves applying inverse dictionary transformation to each current representation, applying a point spread function, and applying the dictionary transformation to each current representation. The iterative process then proceeds to calculate residual error from reference representations and the PSF representation. The current representations are corrected based on the calculated residuals. Thresholding is then applied, and the iterative process returns to the step of applying a point spread function above.

In accordance with the embodiment of Figure 14C, a reference representation is first constructed for each component of a signal for each frame. Then, a current representation is initialized for each component of the signal for each frame. Independent sub-band dictionary transformation is then applied to each current representation and/or each reference representation to create sub-band representations. An iterative process then begins by applying a sub-band point spread function to each current sub-band representation to create a PSF sub-band representation. The residual error is then calculated from sub-band reference representations and the PSF sub-band representation. The current sub-band representations are then corrected based on calculated residuals. Thresholding is applied, and the iterative process returns to the step of applying the sub-band point spread function above. After the iterative process, inverse sub-band dictionary transformation is applied to independent sub-bands and the overall result is output.

In accordance with the embodiment of Figure 14D, a reference representation is first constructed for each component of a signal for each frame. Then, a current representation is initialized for each component of the signal for each frame. A dictionary transformation is then applied to each current representation and/or reference representation. Then, an iterative process begins by applying a point spread function to each current representation to create a PSF representation. The iterative process then proceeds to calculate residual error from reference representations and the PSF representation. The current representations are corrected based on the calculated residuals. Dictionary transformation is applied to each current representation. Thresholding is applied, an inverse dictionary transformation is applied to each current representation, and the iterative process returns to the step of applying a point spread function above.

Thus, in an embodiment, a system comprises: a) an energy source configured to be deliver electromagnetic energy to a volume of tissue; b) a probe configured with features to produce at least one acoustic wavefront directed to propagate into the volume originating at the interface of the probe and the surface of the volume as a direct or indirect result of absorption of the electromagnetic energy by portions of the volume, probe, or interface; c) a transducer array for recording acoustic signals resulting from: i) DAR from electromagnetically absorbent targets within the volume; and ii) SAR from sources of acoustically reflective targets that backscatter (i.e. reflect) from the acoustic wavefront; d) a processing subsystem, comprising: i) a module for simulating acoustic signals that may be produced on delivering the electromagnetic energy to the volume, comprising: 1) a sub-module for simulating DAR signals from the electromagnetically absorbent targets within the volume; 2) a sub-module for simulating SAR signals from the acoustically reflective targets in the volume; ii) a module for reconstructing acoustic signals to produce spatial representations representing the volume, comprising: 1) a sub-module for reconstructing the electromagnetically absorbent targets in the volume; 2) a sub-module for reconstructing acoustically reflective targets in the volume; iii) a module for component separation, comprising: 1) a sub-module for computing a residual between a simulated estimate of the electromagnetically absorbent targets within the volume and a reference based on the recorded DAR signals; 2) a sub-module for computing a residual between a simulated estimate of acoustically reflective targets in the volume based on the recorded SAR signals; 3) a sub-module for modifying the estimates of the targets based on the residuals; 4) a sub-module for outputting final estimates of the spatial representations of (or acoustic signals produced by) the targets.

In an embodiment, the module for component separation is configured to execute a process for component separation, comprising the steps of: a) producing reference representations for DAR and SAR by reconstructing the recorded acoustic return signals; b) computing at least one iteration comprising the steps of: i) applying a point spread function to the current estimates of DAR and SAR by the steps of: 1) simulating the current DAR estimate to produce a DAR sinogram; 2) simulating the current SAR estimate to produce a SAR sinogram; 3) adding DAR sinogram to the SAR sinogram to produce an overall sinogram; 4) reconstructing the DAR from the overall sinogram to produce a DAR PSF representation; 5) reconstructing the SAR from overall sinogram to produce a SAR PSF representation; ii) computing the residuals between the reference and psf representations; iii) multiplying the residuals by a weight to give the weighted residuals; iv) adding the weighted residuals to the current estimates of DAR and SAR; and v) applying thresholding to produce the next estimates of DAR and SAR.

a. Measuring and Processing with the Upward Directed Skin Response

In an embodiment, the volume comprises layered skin tissue and the different skin layers have different optical absorption and/or produce wavefronts of different intensities. The skin layers and properties can vary from subject to subject. The DAR from the skin and coupling layers are amongst the first signals to reach the transducers. Wavefront from the skin layer absorption travel downward into the tissue as well as upward to the transducer. To visualize this phenomenon, consider a point source in a volume that emits a spherical ripple where part of the ripple wavefront moves towards the detector and the opposite part moves away from the detector. Similarly, a planar shaped source will have an upward moving component that reaches a detector and a downward moving component that does not. Hence, the downward wavefront from the skin layer may produce a reflected SAR response from the volume that will correlate with the upward wavefront produced by the skin layer. In an embodiment, the upward moving component is an upward directed response, and the downward moving component is a downward directed response. The wavefront intensities produced by the skin layers are a function dependent on depth. In an embodiment, this can be presented by a 1D function. In an embodiment, the DAR of the skin layers may be detected an analyzed, and used to deconvolve, detect or separate the corresponding SAR signals with methods described herein. For example, if the skin has three layers, three planar shaped wavefronts may propagate upward to the transducers as DAR signals and also downward into the tissue and then reflect back to the transducers as SAR signal. In an embodiment, the skin DAR is first analyzed and may be used directly or may otherwise be used to produce an auxiliary signal that will be expected to characterize the reflections and then used to process or separate the SAR signals. In an embodiment, a 1D skin function is determined by averaging skin signals from each channel, and/or by determining their most prominent component. In an embodiment, the skin function may be determined by extracting this information from a reconstructed image rather than from a sinogram. Hence, in an embodiment, information about the downward propagating wavefront can be inferred or measured from the upward propagating waves, and then used to analyze backscatter of the downward propagating wavefront. In an embodiment, the skin DAR or auxiliary signal is used to form a transfer function, and the transfer function is applied as filtering in the simulation and/or reconstruction modules.

b. Simulation of Probe Features

In an embodiment, a cause of all or part of the SAR signal component can be modeled and the model used to separate such component from the DAR. In an embodiment, a wavefront is caused by a feature or element on or in a probe that delivers electromagnetic energy. A pattern or code can be simulated by treating each feature or element as an independent source (i.e. treating source wavefront elements of a complicated wavefront geometry separately). The backscatter pattern from a point source is easy to model in an ideal case. Any source can be built out of multiple point sources. A line source, cylindrical source, or finite length line or cylindrical source can also be modelled. These sources can propagate due to acoustic mismatch of the probe with the volumetric illuminated background initial pressure source, which is described further below. Also, these sources could occur directly due to initial pressure due to electromagnetic absorption. Wavefront producing features of a probe may make the wavefront, which is substantially unpredictable due to subject variability, more predictable, or may permit the acoustic backscatter from a target to be easier to pinpoint. In an embodiment, features may cause stronger acoustic backscatter. In an embodiment, the produced acoustic backscatter has better convergence when starting with initial conditions in an iterative component separation method.

In an embodiment, only the significant features or elements need be modeled. In other embodiments, complex scenarios are modeled. For example, the surface of the volume and the probe can be represented by a 3D source producing matrix. In an embodiment, each source is broken down (if necessary) into point source elements. In an embodiment, for simplicity spherical wave point sources are used. In an embodiment, the mathematical technique known as Green's function solutions can be used. In an embodiment, a directionality apodization can be applied. In an embodiment, the dot product with a normal is efficient as a directional apodization. In an embodiment, the source strength can be efficiently multiplied as a function of distance. In an embodiment, the source acts on a target as a delta function based on the distance away from the target, and the time elapsed. In an embodiment, the temporal signal received from a target is modeled as the delta function times a magnitude applied to a convolution kernel. In an embodiment, the convolution kernel for an optically absorbing target (simple) is different from a convolution kernel used from a target produced by a mismatched surface reflection due to volumetric illumination (not as simple unless using an approximation). In an embodiment, homogenous speed of sound is modeled in tissue.

In an embodiment, spherical wave point sources are used for simplicity and the signal's intensity is attenuated as a function of distance travelled based on a Green's function solution. Also for illustrative purposes, in an embodiment, a sparse 64x32x8 matrix of sources is used to model the wavefront resulting from the probe. The aspect ratio of the voxels can be substantially equal, so the voxels are cubic voxels, or each voxel represents a point source. Dimensions of the probe face for this example are 40mm x 20 mm x 0.5mm. In this example, the air surface outside of the probe is not modeled using this matrix, but this can be modeled by adding an overall ideal plane wave convolved with a kernel that is a function of depth, or for simplicity a constant kernel. All of the voxels where z=1 in the probe to can be set to 1.0. For voxels beneath the optical window, these voxels where z=1 can be set to 2.0 and where z=32 to - 10.0 (to simulate a 3D coded feature). A random coded pattern can be placed on the surface of the probe to correspond to random small beads located on the probe at the grid sites determined to randomly contain a bead. Thus, in a constructed probe, which grid sites should contain a bead may be randomly determined, and in the event that a bead is present, a bead will be placed on the probe in the corresponding spot. For illustrative purposes, the bead will be a strong source, so when a bead is present, the value of 20.0 is added to the 3D matrix where z=1. For this example, in an embodiment, 40 beads are placed at random positions on the grid of the probe face, but not on top of positions corresponding to the glass window and not on top of regions near transducers. There will be an ideal acoustical isolator surrounding the detector elements that does not reflect acoustic signal. The embodiment will also include a source of value 5.0 to correspond with the position of isolator at z=1. If incident wavefront produced by this 3D matrix of sources is simulated, each point in the tissue will receive a different time domain wavefront signal. The strongest features from the matrix will be received by the point in the tissue. For the moment, angular dependence is ignored. The SAR signal will be based on acoustic reflections of the wavefronts as sent to the tissue by the probe, according to the time domain wavefront signal, which in general will be different at each position in the tissue, especially for points that are not nearby each other. Points that are close by may experience a similar time domain wavefront. In an embodiment, the time domain signal for each point will be a summation of each source intensity in the 3D matrix, occurring at a time related to the propagation delay from the matrix position to the point, and a weighting of the source in proportion to the propagation delay and as a function of the angle. By examining the time signals seen at a point in tissue due to just the beads, and ignoring magnitude of the intensities, then the time signal from the beads will consist of an impulse corresponding to each bead based on the propagation delay to the position and the bead.

Since attenuation of this received signal will be a decreasing function of distance, in an embodiment, the magnitude of the impulses based on a simple decreasing function of distance in the time domain can be modeled. If the situation is highly non-ideal, then in an embodiment, the results will be approximate, causing errors in the time domain signal, thus sacrificing resolution. In an embodiment, the wavefront from acoustic mismatch due to volumetric illumination can be modeled as a non-stationary convolution with depth, or an approximation of a stationary convolution can be used. In an embodiment, edges or line sources can be modeled as point sources convolved with a suitable waveform, and added under superposition. In an embodiment, each point in the tissue has a one-dimensional filter corresponding to the coded impulse response in the time domain. In an embodiment the filter has a corresponding wiener deconvolution filter. In an embodiment, as a simplification, the filter for each point in the tissue can be common for all detectors. In an embodiment, if a code pattern is only a function of one spatial parameter, such as depth, there can be a common filter for all points of equal depth. In an embodiment, the features can produce a code pattern that is approximately separable in more than one spatial coordinate, and the filter can be a composition of this separability.

In an embodiment, a backscattered signal from a volume is spatially coded by embedding features or elements on the probe (or other system component) to independently modulate each spatial position of the tissue with a foreknown time domain waveform, resulting in a superposition of backscatter caused by each element or feature. When the acoustic signal from all receivers is measured, and beamformed to a particular spatial position (by applying delays) the time-domain beamformed signal will (instead of being a delta function from the backscatter) be modulated according to the acoustic reflections caused by the features on the probe. Since it is known in advance what code or response has made its way to each position, the resulting time domain signal can be correlated with the known code or response that had reached a position. Deconvolution can be used to determine the signal arising from the code or response. Hence, deconvolution that makes use of the features on the probe that cause this effect can be compensated advantageously. Stated another way, DAR signals will not be correlated with patterns from the probe features, but PAB signals will be correlated with the pattern of probe features. Hence, correlating wavefront backscatter with waveforms based on wavefront producing features of the probe permits separation of the DAR signal from the PAB signal. It also helps identify reflective targets for unpredictable wavefronts resulting from subject variability, since predictable wavefronts are used to mark the reflective targets with a predictable signature.

In an embodiment, a wavefront of a distinctive nature propagating into the tissue can be used. Such a wavefront may be appropriate even where a similar code waveform will reach all positions in the tissue. Computationally, it may be easier to separate DAR signals from wavefront PAB signals if all wavefront backscatter sources are modulated with a similar code. In an embodiment, the edges of the probe from the air-tissue-skin boundaries can serve as features that may be used to distinguish between DAR and SAR, and thus helpful to separate at least one of them. The code waveform may change slowly as a function of depth. In an embodiment, an optical exit port of the probe may produce wavefronts that may be used to aid in distinguishing between DAR and SAR signals, and thus helpful to separate at least one of them. In an embodiment, other features of the probe surface may produce wavefronts that may be useful to separate DAR from SAR signals.

When the DAR signal and SAR signal are highly correlated, they may be difficult to distinguish and thus, to separate. By identifying features of the probe that cause a known incident wavefront, differences between the return signal and backscatter signal information can be more easily identified. Similarly, by using features of the probe to control the incident wavefront, the correlation between the return signal and backscatter signal information can be reduced, leading to an improvement in component separation and/or SNR.

In an embodiment, known wavefront sources external to the volume may be simulated to determine wavefronts that will propagate into the volume. In an embodiment, wavefront sources that arise from targets within the volume (e.g., vessels) may be simulated to determine wavefronts that propagate within the volume. In an embodiment, a map may be created to represent the temporal impulse response waveforms reaching different locations of the volume due to wavefronts from optically absorbing sources within and/or external to the volume. In an embodiment, a DAR spatial representation may be used to represent optically absorbing sources external to, or within the volume. In an embodiment, initial pressure sources may be used to determine maps of waves in the volume at numerous time-steps. In an embodiment, spatially dependent temporal impulse responses may be extracted from maps of waves in the volume at numerous time-steps because the temporal impulse response is related to the pressure waves arriving at a position as a function of time. In an embodiment, the simulation of SAR may apply temporal impulse responses to corresponding (e.g. proximate) acoustically reflective targets when totaling the contribution of these targets to the sinogram. An omnidirectional assumption may be used in such totaling, and/or during wavefront simulation.

In an embodiment, the acoustic waveform from an absorbing 1D spatial pattern (i.e. a line) on the surface of the probe that reaches a target in the volume will vary as a function of position. Consider a 1D absorbing pattern defined by the function f(r) placed on the surface of the probe along the line defined by: (r * cos(theta), r * sin(theta), 0). Assuming a homogeneous medium with sound speed c, then at time t, the portion of the acoustic wave that reaches position (px, py, pz) in the volume will correspond to f (px*cos(theta)+py*sin(theta) + sqrt((px^2 -py^2) * cos(theta)^2 + 2 * cos(theta) *px*py*sin(theta) - pz^2-px^2+t^2*c^2)). That is to say, that the portion of the 1D pattern responsible for the acoustic waveform reaching the position will be temporally distorted by constants C1, C2 and C2 as f(C1+sqrt(t^2*c^2-C3)) that change with regard to position in the volume and orientation of the line. Hence, in an embodiment, the 1D pattern can be used to spatially encode the volume according to the known constants. In an embodiment, the pattern on the line can be broken down into point sources and the solution for the acoustic waveform reaching positions in the volume can be determined using Green's function methods. In an embodiment, multiple 1D line patterns can be used in superposition (e.g. an "X" shape). In an embodiment, when an absorbing 2D pattern on the surface of the probe produces an initial pressure distribution, frequency domain methods can be used efficiently for solving the acoustic waveform reaching positions in the volume. In an embodiment, to compute the waveforms in the volume from a 2D surface pattern, existing methods for computing signals reaching 2D planar detectors from a 3D volume can be adapted by using temporal reversal with a Dirac impulse applied to the time domain input corresponding to the illumination. In an embodiment, a simplification of this adaptation yields a fast solution for signals in an imaging plane.

In an embodiment, when the known produced waveforms at positions in the volume, as described above, are sufficiently unique at different positions in the volume, the backscatter from each of the different positions that will be recorded by the transducers can be said to contain a signature sufficiently unique to encode the different positions in the volume. In an embodiment, fronts of the produced wavefronts reach targeted positions in the volume. In an embodiment, the fronts that are seen by targets at the targeted positions are known (i.e. substantially deterministic) produced time-domain waveforms. Thus, in an embodiment, backscatter received from a position in the volume will, in a manner, be modulated with a spatially varying code. For example, in a situation where two positions are equidistant from a transducer element, a different spatially varying code would correspond to each position. The backscatter signal received by the transducer element from the first position would interfere with the signal received by the transducer element from the second position. However, in an embodiment, the intensity of a signal component corresponding to the first code and the intensity of a signal component corresponding to the second code can both be computed as a way to quantify the intensity of backscatter at each position, thereby discriminating between the two interfering components of the signal. In an embodiment, in a dense volume the intensity of signal components corresponding to each position can be computed. In an embodiment, multiple transducer elements are used. In an embodiment, an iterative method (e.g. an iterative separation method) is used to determine the backscatter intensity from multiple interfering positions in a volume. In an embodiment, spatially varying wavefronts encoding a volume are used to discriminate between signal components received from sources equidistant to a single transducer element. In an embodiment, spatially varying wavefronts encoding a volume are used to discriminate between signal components received from sources at varying elevational angles when the sources are equidistant to an axis of a 1D transducer array. In an embodiment, the volume is considered a linear system, and frequency content of incident acoustic wavefronts penetrating the volume will produce acoustic backscatter components with substantially the same frequency components as the incident wavefronts. In an embodiment, incident acoustic wavefronts with controlled frequency contents can be directed into the volume and used to identify the acoustic backscatter component.

OPTO-ACOUSTIC ISOLATORS

In an embodiment, the probe incorporates an isolator that reduces the amount of energy received by one or more acoustic receivers. In an exemplary embodiment, the isolator is an opto-acoustic isolator that reduces the amount of energy transmitted from a light path of the probe to a transducer assembly, which is also positioned on or near the probe. Such an isolator is described in US20140206978, the isolator substantially reduces one or more artifacts in images reconstructed from acoustic signals received by the probe. In an embodiment, the isolator absorbs acoustic waves. It may be fabricated, for example, from a material with a high acoustic attenuation coefficient across a broad range of frequencies. In an embodiment, the isolator does not reflect acoustic waves originating from the volume back into the volume. In an embodiment, the isolator produces a wavefront that will reflect off of acoustically reflective targets in the volume as a SAR signal. The isolator can be located for producing wavefronts at a suitable position on the probe surface or other system component. In an embodiment, an isolator on the surface of the probe may be coated partially or fully with an optically reflective coating. In an embodiment, when the isolator is coated with an optically reflective material, a wavefront from optical absorption is not produced or is substantially reduced. In an embodiment, the isolator may be colored with an optically absorbing coloring, which may reduce optical energy penetrating the probe. In an embodiment, the isolator may be colored with an optically reflective coloring, which may reduce optical energy penetrating the probe. In an embodiment, when the isolator is colored with an optically reflective coloring, a wavefront is not produced from optical absorption or it is substantially reduced. In an embodiment, the isolator and surrounding portions of the probe surface may be covered with a pattern. In an embodiment, horizontal or vertical features cover the isolator, such as bars, lines or a rectangle on the distal surface of the probe. In an embodiment, when such features lie parallel to an array of acoustic receivers, stripe filtering may be applied to a sinogram to reduce any interference caused by such features. In an embodiment, the light reflective coating is gold or gold paint, a metal or metallic paint, or other such suitable coating. In an embodiment, the wavefront producing feature is an uncoated isolator. In an embodiment, a parylene coating is used in the isolator. In an embodiment, a spacer is used in lieu of an isolator. In an embodiment, the isolator can reduce SAR and/or PAB artifacts in images reconstructed from received acoustic signals. The isolator or other components (e.g., a spacer, a probe and an optical window) can be modified in accordance with the present disclosure to control the wavefronts produced by optical absorption and/or acoustic reflection, such as, for example, to increase the intensity of the wavefronts, decrease the intensity of the wavefronts, or make patterned wavefronts. In an embodiment, the optical absorption of an isolator alters the fluence distribution in the imaging plane, which may also reduce near field artifacts. Optical absorption occurring on the surface of the isolator can reduce the light delivered to the near field directly beneath the transducer assembly, which can reduce first order ringing and reduce downward directed wavefronts impacting the imaging plane below the transducer assembly that occurs due to the mismatch between the volume and the transducer assembly and due to the high skin absorption. Hence, it is believed that having an isolator with high optical absorption may transfer the energy of downward directed wavefronts and artifacts associated with high near field illumination from the imaging plane to wavefronts originating adjacent to (away from) the imaging plane, which improve visibility in the near and mid fields. In an embodiment, the externally exposed isolator surface forms a rectangular shape with an interior rectangular shape for the transducer array, such that the boundary can be grouped into four bar shaped feature segments. In an embodiment, enhanced coating of the isolator should further reduce artifacts. In an embodiment, the other methods described herein may further reduce artifacts by separating signal components that occur as a result of this effect.

Sparseness in the Component Domain

In an embodiment, the reconstructions for DAR and SAR will tend to be more sparse in the appropriately reconstructed domain. For example, a SAR signal from an acoustically reflective target will have a tendency to be represented more sparsely in the SAR reconstructed image domain than in the DAR reconstructed image domain. Correspondingly, a DAR signal from an electromagnetically absorbent target will tend to be represented more sparsely in the DAR reconstructed image domain than in the SAR reconstructed image domain. In a DAR reconstructed image, an acoustically reflective target will be smeared. See, for example, the DAR reconstructed images in Figures 10a and 11a. In an SAR reconstructed image, an electromagnetically absorbent target will be smeared. See, for example, the SAR reconstructed images in figures 10e and 11e. This sparsity allows the processing system to effectively separate the signal. In the sinogram domain, a point target is not localized to a point, thus it is not represented localized in the sinogram; rather a point target is represented as a curve in the sinogram. Thus, in a preferred embodiment, the sparsity of the reconstructed image domain is used as a minimization constraint. As targets tend to be contiguous, they will also be sparse in other domains. Thus, in an embodiment, maximum sparseness can be obtained in the appropriately reconstructed image domain for the component that further transformed into an additional sparse basis.

Using SAR to Indicate Regions of Tissue

In an embodiment, weakly scattering tissue will permit an incident wavefront to travel, while strongly reflecting tissue, such as e.g., lung tissue, will reflect substantially an entire incident wavefront. In an embodiment, using the teachings disclosed herein, detection of a reflected wavefront from lung or similar tissue and separation of this SAR signal from DAR is performed. In an embodiment, the SAR signal from lung or other such tissue can be detected, and used to mark or delineate the position of this tissue in an image. In such case, signals from depths beneath the lung tissue can be lessened or removed from an OA image. For example, lung tissue causes a strong reflection (as shown in Figure 7). Even in cases when the component separation is not perfect, the detection of a strong separated signal or with strong characteristics can signify that the portions of the DAR image (e.g., beneath the delineated SAR target) should be completely weakened or deleted, even though the SAR signal has not been completely separated. For example, in Figure 7, reconstruction of the SAR component (not shown) may yield a contour of high intensity that lines-up with the strongly reflecting boundary in the ultrasound image. In an embodiment, the SAR signal is used to detect or segment regions of the DAR signal or DAR image that should be mitigated, not displayed, or displayed separately. In an embodiment, a user can indicate (by drawing a line, moving an indicator, or other input) a non-imaging region or depth containing lung, bone, muscle, or other interfering tissue. In an embodiment, this indication is used to mitigate an unwanted signal. In an embodiment, this indication is used in combination with component separation to mitigate the unwanted signal. In an embodiment, the presence of a strong reflection from the separated SAR signal is used to automatically segment, characterize, or delineate unwanted regions of the image. For example, in breast imaging, lung tissue may have a strong reflection, that would otherwise not be present, and would be much stronger than in other breast tissue, hence the SAR signal or SAR image can be used to indicate the boundary of this region (even when the component separation is not completely effective and even where only a first pass reconstruction for the SAR image has been computed). In an embodiment, segmentation is performed on the SAR image to determine where the regions of tissue, if present, are located; following this, unwanted regions of the image (e.g., the lung tissue), if detected, may be removed from the image or from a sinogram. In an embodiment, an algorithm to perform the mitigation is provided comprising: i) when the overall SAR component in the SAR image matches a prescribed criteria then, ii) for each pixel coordinate along the horizontal axis, iii) find the shallowest vertical depth pixel in the SAR image that has intensity beyond a given level; iv) next, if such a pixel was found, then zero out all pixels in the DAR image at the current horizontal coordinate from substantially the found vertical depth and deeper; v) repeat from step iii) for the next horizontal coordinate. In an embodiment, the prescribed criteria may include the presence of a strong SAR ridge segment in the SAR image, such as a ridge that may be present from lung or rib tissue. In an embodiment, the criteria may include where the normalized overall intensity of the SAR image is greater than a prescribed level.

Out-of-plane Structures

In an embodiment, out-of-plane structures can be detected and identified with the coded waveform. In an embodiment, the probe may produce an incident wavefront designed to differentiate backscatter in from objects passing through imaging plane from out of plane objects. In an embodiment, iterative minimization is used to reconstruct a 3D spatial representation of a volume using sinogram measurements with a 1D transducer array, which can determine out of plane structures as described above.

Vessel Detection

In an embodiment, optically absorbing targets that are strongest and/or conform to a specific shape profile in a reconstructed image may be assumed as vessels. In an embodiment, assumed vessels are automatically detected. In an embodiment, vessel detection involves finding regions of an image containing a shape profile, e.g. by correlating with a shape profile filter. In an embodiment, a shape profile filter may detect ridges, hyperbolas, arcs, curves, blobs, lines or other such shapes. The shape profile of a vessel and/or cylindrical object may depend on its position relative to the probe and on its orientation (e.g. polar and azimuth angles) when crossing the imaging plane. The depth of a target represented in an image is related to its distance from the probe. Commonly, a vessel crossing the imaging plane will be at a closest distance to the probe where it intersects the imaging plane. When an illustrative marker touching a vessel is moved away from the imaging plane, the distance of the marker to the probe may increase. Consequentially, portions of a straight vessel may appear to bend deeper in an image as portions of the vessel extend away from the imaging plane. Accordingly, characteristic streaks may be observed from vessels in an image. Since this bending or streaking depends on the position and orientation of the vessel, in an embodiment, orientation and/or position may be extracted (i.e., deduced) from an image or data that captures a vessel or other such object. In an embodiment, the crossing of an object through the imaging plane is represented by template curves for different positions and orientations. In an embodiment, the data and/or image representation of a target object is matched to the template curves to determine orientation and/or position. In an embodiment, the template curves may follow an equation, be extracted from simulation, or obtained otherwise to describe how an oriented object is expected to appear. In an embodiment, a polar angle, and azimuth angle and/or a position of the object with respect to a co-ordinate reference (or other such angular representation) is output. In an embodiment, the position is used as an input and the orientation is an output. In an embodiment, the path of the vessel or object is traced in the image or sinogram, and the traced path is best fit onto a curve (e.g. that represents a parametric equation describing orientation and/or position) such that the best fit solution yields the sought orientation and/or position.

In an embodiment, the volume is spatially represented by coefficients in a dictionary, basis or frame of steerable wavelets. Steerable wavelets allow, for example, ridge elements or steered ridge detection filters to be represented by a small number of independent coefficients whereby the steering orientation can be efficiently extracted from the coefficients. In an embodiment, when a volume is represented by steerable coefficients, iterative reconstruction or similar methods can be used to find a sparse solution for representing the volume in the dictionary of the coefficients. In an embodiment, in any such sparse representation of the volume by steerable coefficients, the strongest and/or non-zero magnitude indices can represent the structures (e.g. vessels) of interest, and the orientations can be extracted. In an embodiment, a 2D imaging plane is represented by coefficients of 3D steerable structures. In an embodiment, a 3D spatial representation is converted between a 3D steerable wavelet representation during reconstruction and simulation operations. In an embodiment, 3D steerable coefficients are found from a 3D wavelet representation of the volume by applying directional derivatives and the inverse square-root Laplacian operation or an approximation thereof. In an embodiment, the 3D representation of the volume can be used to remove streaking artifact of vessels crossing the imaging plane. In an embodiment, vessels are automatically detected using this method. In an embodiment, an image of the detected vessels is formed and is displayed overlayed on top of another image. In an embodiment, multiple wavelengths can be used in such detection as described herein. In an embodiment, only the oxygenation and/or hemoglobin levels of such detected vessels are displayed. In an embodiment, the detected vessels are converted to a data structure used to represent a vascular tree, vascular network or vascular segments. In an embodiment, the vascular tree representing data structure is used to improve motion tracking when motion is present between acquired frames. In this manner, determining the position of a vessel as it appears in two adjacent frames is possible, because a slight position or orientation offset can be tracked and accounted for, thus ensuring that a detected object corresponds to the same vessel. The represented vessels may provide useful structures for a motion tracking algorithm to lock onto. In an embodiment, the represented vessels (e.g. vascular segments) are assumed, to a first order, to follow a straight path, such that when a small motion is undergone by the probe, the position of a vessel in an adjacent frame is slightly shifted according to this approximated straight path followed by the vessel. For example, if a vessel follows the path of a line, and the imaging plane remains parallel in an adjacent frame, the position of the vessel in one frame compared to its adjacent frame can be visualized as a line intersecting two parallel planes, and the orientation of the vessel in each plane will correspond to the slope of the line. In an embodiment, the shift in position of a vessel of given orientation that is not parallel to the motion can be used to estimate the speed of the motion when the duration between the acquired frames is taken into account. In an embodiment, the vessels or vessel segments are represented as lines or line segments. In an embodiment, a vessel has a vessel configuration with parameters such as position and/or orientation. In an embodiment, an acquired frame is represented as a reference plane and an adjacently acquired frame is represented as a plane with an unknown configuration (e.g. position and orientation) that intersects the lines (or line segments) representing the vessels. In an embodiment, the unknown configuration is solved by finding a configuration that minimizes the sum of errors (e.g. distances) between the mapped position of each detected vessel in the adjacently acquired frame (when mapped through a transformation from the reference plane to the configuration of the unknown plane) to the intersection of the line representing the vessel and the unknown plane. In an embodiment, this can be solved by minimizing a linear program.

In an embodiment, the affine transformations (e.g. undergone by a probe) between such locked onto structures can be determined. In an embodiment, when orientations of substantially all detected vessels (or other such targets) between adjacent frames are subjected to the same (on average) affine transformation, this substantially reveals the motion undergone by a probe, and the motion may be extracted by solving it from a determined overall affine transformation subject to the constraints of rigid motion. In an embodiment, if the orientations of the vessels remains constant, the motion of the probe is parallel. In an embodiment, the solved transformation is a best-fit solution of the motion undergone by the probe. In an embodiment, the solved transformation must be adapted to produce the motion undergone by the probe (e.g. using a coordinate transformation). In an embodiment, the affine transformation is a linear transformation or a coordinate transformation. In an embodiment, the location of an unknown plane that intersects lines representing the vessels is solved to find the motion of the probe. In an embodiment, non-rigid tissue deformation has also occurred, and this can be solved by computing a difference between the affine transformation found for each vessel (or target) and the overall affine transformation, and substantially using interpolation to determine the deformation map for the remainder of volume representation. In an embodiment, when no vessel structures are present, correlation analysis between tissue regions of adjacent frames can be used for freehand motion tracking.

c. Output/Storage Device

Figure 8b is a block diagram showing an overall component separation process. In an embodiment, an output module is provided capable of outputting one or more spatial representations or acoustic signals in a manner that they can be viewed, stored, passed, or analyzed by a user or other analysis module. In an embodiment, unrefined spatial representations reconstructed from recorded acoustic signals are displayed or output. In an embodiment, spatial representations are displayed or otherwise output after application of additional image processing. In an embodiment, intermediate spatial representations are output or displayed. In an embodiment, refined spatial representations are output or displayed. In an embodiment, reference DAR and SAR spatial representations are displayed or otherwise output. See, for example, Figures 10a, 10e, 11a, and 11e. In an embodiment, PSF spatial representations are output or displayed. See, for example, Figures 10d, 10h, 11d, and 11h. In an embodiment, component separated spatial representations are output or displayed with or without thresholding. See, for example, Figures 10b, c, f, g, 11b, c, f, g. In an embodiment, only the DAR representation and not the SAR representation is output or displayed, in which case the SAR representation may be discarded. In an embodiment, signal domain DAR or SAR are output or displayed, which may be computed by applying the simulation module to the spatial representation. In an embodiment, processed representations of DAR or SAR are output or displayed as shown in Figure 8b.

B. Surface wave separation

An acoustic signal and the resulting sinogram may also contain an acoustic surface wave (ASW) signal. In an embodiment, the method of component separation described above, can be adapted to include the separation or removal of the surface wave component from acoustic signals. In an embodiment, this can be done with our without separation of the SAR component. Thus, in an embodiment, a DAR component is separated from an ASW component. In other embodiments, an ASW component is separated from an SAR component, with or without separation of the DAR component. In an embodiment, no significant wavefront is produced; and thus, there is no SAR component to remove.

In an embodiment, surface waves are modelled as point sources originating on a plane parallel to the probe's (or other system component's) surface, or following the surface of the tissue. In an embodiment, features of the probe (or other system component) may produce acoustic surface waves. Surface waves travelling along the surface of the probe can remain detectable even when the probe (or other system component) is not in contact with the volume. Such surface waves may change when the probe comes into contact with the volume. In an embodiment, this change may be used to detect when the probe comes into contact with the volume. In an embodiment, these surface waves may be modelled and separated. In an embodiment, surface waves may cause backscatter, when they reflect off features on the surface, or in the volume. The same methods described above for removing an SAR signal, can be applied to removal of an ASW signal, wherein the simulation and reconstruction are modified to simulate and reconstruct the surface waves rather than the DAR or SAR signals.

In an embodiment, first order surface waves from the probe features reach the acoustic receivers first. If the probe has a different speed of sound than the volume or a gel or other coupling medium used between the probe and the volume, then a wavefront propagating along the probe will reach the receivers in a different timeframe than the wavefront travelling along the surface of the volume or through the coupling medium. In an embodiment ASW may include mechanical waves travelling along the surface of the probe, the surface of the volume and/or through the coupling medium. Measuring the differences in arrival times of the signals can provide valuable information about the coupling. As the arrival times may be different for the waves travelling along the surface of the probe, the surface of the volume, and through the coupling medium, this implies that the speed of sound (e.g. shear or longitudinal) of each material is different. Thus, in an embodiment, this can be measured. In an embodiment, the differences in arrival times (or delays) are used to separate signal components as discussed above.

In an embodiment, if the features are horizontal or vertical to the detector elements, the surface waves will either reach all elements at the same time for parallel, or sequentially propagating to create a diagonal line in the sinogram. In an embodiment, stripe filtering can be used to remove such waves from the DAR component of a sinogram. In an embodiment, when the probe and the volume are coupled together, they are also surrounded by air, which is a configuration that may produce a surface wavefront resulting from a discontinuity at the boundary of the probe surface (as described in more detail below). In an embodiment, such a wavefront propagates sequentially to detector elements in an array (e.g. creating a diagonal line in a sinogram). In an embodiment, such a wavefront can be used, as described above, to infer information about the coupling interface (e.g. velocity or speed of sound of materials, status of coupling, thickness of coupling medium). In an embodiment, if the probe is partially coupled to the volume and partially exposed to air, this situation can be detected, and the position of where the coupling is lost can be determined. In an embodiment, the slope of a produced diagonal line in the sinogram is proportional the speed of sound of a surface wave, and thus can be used to measure it. In an embodiment, if the wave travels with different speeds, the observed diagonal line disperses. In an embodiment, when this occurs, the line fans out (e.g. an elongated triangle). In an embodiment, the intersection of a diagonal line in a sinogram with the time zero intercept indicates the position on the probe surface where the wavefront originated. In an embodiment, the intensity of the produce signal yields information about the coupling interface (e.g. acoustic impedances). In an embodiment, the change in intensity of the measured surface wave varying at sequential detector elements yields information (e.g. acoustic attenuation properties). In an embodiment, an opto-acoustic image is formed that uses at least one parameter computed from measuring an observed surface wave in the sinogram.

In an embodiment, an acoustic isolator can be used to mitigate shear waves, elastic waves or other such waves that would propagate internal to the probe, and in particular that can occur due to energy from the light path reaching the acoustic receivers. Thus, in an embodiment, when an isolator is used, the ASW component from features is assumed to have traveled proximate to the probe surface. In an embodiment, the isolator may reduce ASW surface wave component.

Parametric maps can be computed using the methods described in WO2013056089.

In an embodiment, a single light source is used, the single light source delivering light (or other electromagnetic energy) to a volume of tissue at a single wavelength - or within a very narrow band of wavelengths. In an embodiment, multiple light (or energy) sources are used, each being able to deliver electromagnetic energy to a volume at a narrow band or single wavelength. In an embodiment, light is delivered through the distal end of a probe that may be positioned proximate to the volume. In an embodiment, the light is delivered via a light path from the light source to the distal end of the probe. The light path may include fiber optic cables or other transmission means. The light path may include one or more light exit ports, and may also comprise one or more lenses, one or more diffusers, and/or other optical elements.

In an embodiment, the light source comprises a tunable laser capable of delivering light to the volume at different predominant wavelengths at different times. In an embodiment, the light source delivers multiple wavelengths of light at the same time (i.e., having multiple narrow bands of light in a single light pulse). In an embodiment, multiple light sources are used, each having its own light path. In an embodiment, the light paths overlap in whole or in part. In an embodiment, two lasers are used capable of delivering pulses of light at different predominant wavelengths. In an embodiment, an NdYAG laser capable of emitting a wavelength of around 1064nm and alexandrite laser capable of emitting a wavelength of around 757nm are used. In an embodiment, the light source for producing light at or near a predominant wavelength is selected from the group consisting of a laser diode, a LED, a laser diode array, and a pulsed direct diode array.

In an embodiment, the system comprises one or more receivers for receiving the resulting acoustic signals such as the transducer arrays or other receivers described above.

A component separation system and method according to the disclosure in this section further comprises a processing subsystem adapted to analyze the acoustic signals to obtain information regarding electromagnetically absorbent targets in the volume. In an embodiment, the processing subsystem analyzes the acoustic signals to produce a spatial representation of the targets in the volume

C. Types of Wavefronts

Acoustic wavefront(s) can result from various sources. For example, an acoustic wavefront can result when a source in or proximate to the volume absorbs the electromagnetic energy and produces acoustic pressure. Generally this acoustic pressure is the result of the release of temporal stress confinement. In an embodiment, the electromagnetic energy is delivered to the volume via a probe. In an embodiment, the electromagnetic energy may be created by a light source within the probe, or a light source that is fed to the probe (e.g., via a light path). The source of an acoustic wavefront can also be in or on the volume. In an embodiment where the volume is tissue, sources of an acoustic wavefront can include, e.g., a vessel (e.g., a blood vessel) or feature of the epidermis. In addition to being in or on the volume, acoustic wavefronts can also be produced by acoustic energy absorbed or reflecting off of an element, feature, target, material, or other source that is external to the volume. For example, the acoustic energy may reflect off of a reflective element or feature in or on the delivery mechanism for the electromagnetic energy, the acoustic receiver, and/or materials used to house them (e.g., the probe). The reflecting acoustic energy may be caused by background initial pressure resulting from the electromagnetic heating of the volume. An acoustic wavefront can also result from acoustic energy reflecting off an impedance mismatch between materials in or proximate to the volume. For example, the acoustic wavefront can be produced when a portion of a surface of the volume is adjacent to a medium that is not perfectly matched to the acoustic properties of the volume. In an embodiment, electromagnetic energy is delivered to a volume via a probe that is proximate thereto, and an acoustic wavefront originates at the interface between the probe and a surface of the volume. In an embodiment, where the volume is human or animal tissue, an incident wavefront may originate at the surface of the skin. The incident wavefront may be due to an impedance mismatch, the skin-probe interface and/or, in an embodiment, a skin-air interface adjacent to the skin-probe interface. In an embodiment, where the volume is human or animal tissue, an incident wavefront may originate from the epidermal layers of the skin, and/or in or at the surface of a coupling medium positioned on the probe, on or the skin, there between and/or proximate thereto. In an embodiment, the probe may be acoustically mismatched with the volume. In an embodiment, acoustic transmitters or one or more transducers may be used to generate acoustic wavefronts. It will be understood that an incident acoustic wavefront may be partly reflected from a target with weak acoustic scattering such that substantially lower energy is diverted to the reflected wave than is contained by the incident wavefront. Moreover, it will be understood that an acoustic target may also be a wavefront source and vice versa.

Use of the term wavefront here is not intended to imply that it is only the front of the wave that may create SAR or other signal components. Hence, the term wavefront as used here includes a wave that may have a front as well as other parts of the wave (e.g., middle and rear). It is to be understood that any part of the wave may create SAR other signal components. In some circumstances, a wave may have more than one "wavefront."

Wavefronts Induced by Volumetric Illumination

When an opto-acoustic source homogeneously illuminates a half-plane (half space), a planar wave front will propagate. It can be represented as a function of one spatial parameter (e.g. depth). The equation can be derived as:

$$p(x,t) = \begin{cases} \dfrac{1}{2}H(x+ct) + \dfrac{1}{2}H(x-ct), & ct < x \\[2mm] \dfrac{1}{2}H(x+ct) - \dfrac{1}{2}\alpha H(-x-ct), & 0 < x < ct \end{cases}$$

where H is the 1D initial pressure distribution profile, and alpha is the strength of the reflection, x is depth, and p(x,t) is the pressure at depth x, time t, and c is speed of sound, and x>0.

In the situation involving a non-ideal produced wavefront, which may be the result from an opto-acoustic probe, the wavefront may not match an ideal plane wavefront resulting from an illuminated surface, or an ideal reflection resulting from a homogenously illuminated half-plane. Consequentially, in an embodiment, the layout of the probe (possibly including the layout of the acoustic detector, if the backscattered wave can be better inferred from a specific detector layout) must be accounted for. Thus, in an embodiment, consideration should be given in the design of a probe to the incident wavefront that it will produce. In an embodiment, a probe may be designed with an objective of reducing such a probe-caused incident wavefront. In an embodiment, a probe may be designed with an objective of maximizing such a probe-caused incident wavefront. In an embodiment, a probe may be designed with an objective of ensuring consistency across the variability arising in a clinical situation, so that component separation will be reliable. It is within the scope of this disclosure to quantify the effect that the features of a probe have on the generation of wavefronts, and use that information to separate SAR (or other signal components) from DAR. It is also within the scope of this disclosure to purposely configure a probe with features or a pattern to generate a wavefront and use the known wavefront producing features or patterns to separate SAR (or other signal components) from DAR.

Wavefronts from Discontinuities

In the boundaries inside of a tissue volume, when one tissue adjoins with the next tissue, the optical, acoustic, and mechanical properties of the volume may change because two different types of tissues may have different optical, acoustic, and mechanical properties. Taking blood vessels for example, blood vessels have low acoustic contrast compared with surrounding medium, but, because the optical contrast is high, the differences in such properties may not affect opto-acoustic image reconstruction. In an embodiment, such properties are considered substantially correlated. In an embodiment, the properties are treated independently. When the properties are treated independently, the simulation and reconstruction of the DAR may be performed separately from the simulation and reconstruction of the SAR.

For acoustic waves, when a target acting as a source has a spatial boundary (a discontinuity), a wavefront may be emitted from the boundary. When a probe is placed on skin, the edges of the probe can act as boundaries. The tissue-air interface can also act as a boundary. The probe-air interface can also act as a boundary. Acoustic discontinuities can also act as boundaries. In opto-acoustics, sources of DAR are sources of initial pressure resulting from energy absorption. In opto-acoustics, when a source target has a boundary (discontinuity), the resulting source of initial pressure due to the energy absorption will be in the shape of that target. Thus, the boundaries of that target can help to determine the wavefronts. For example, a finite-length cylinder (as opposed to an infinitely long cylinder) has boundaries at the ends of the cylinder (as well as its cylindrical surface). In the ideal infinitely long case, only the cylindrical surface is accounted for. The ends of the cylinder, however, do produce wavefronts that may cause backscatter. The same holds true for the non-infinite contact of the skin with a probe through a coupling medium. For a simplistic probe face illustrated as a rectangle, instead of a large surface, the edges of the rectangle as well as the probe surface may produce wavefronts, and the surrounding air tissue interface may also form a wavefront. In an embodiment, tapering the edge of a probe may help to direct the wavefronts resulting therefrom. In an embodiment, wavefronts may be produced by the surface of the probe, including the transducer assembly, coatings, optical windows (optical exit ports), material discontinuities, the distal surface of probe housing, and the surrounding air (i.e., non-contact region). In an embodiment, a produced incident wavefront carries the acoustic impulse response from the pattern of the surface of the probe to acoustically reflective targets in the volume.

II. Coded Probe

In another aspect of the disclosed methods and apparatus, an element or feature (either on or in a probe or otherwise situated) is added or modified to produce one or more recognizable "artifacts" in resulting acoustic signals or spatial representations. In an embodiment, the recognizable artifact does not distort the DAR image or is substantially imperceptible to a human, but can be recognized by computer processing (e.g., like a digital "watermark"). In an embodiment, the recognizable artifact is perceptible in the image only when a physiological feature of tissue is present (e.g., to identify a cyst, to identify neovascularization, etc.). In an embodiment, the added or modified element or feature produces one or more predictable acoustic wavefronts or resulting waveform patterns. In an embodiment, it can be said that the probe or other component of the system is "patterned" or "coded" to produce the predictable wavefronts or waveforms. The predictable wavefronts or resulting waveform patterns can be described analytically, by simulation, or by experimentation and measurement. The processes and systems described above can then be modified to better isolate an SAR signal caused by the predicted wavefront(s) or waveform(s). For example, a transfer function can be designed to match the predicted wavefront(s) or waveform(s). In an embodiment, an SAR signal is isolated so that it can be removed. In an embodiment, the SAR signal is isolated and used to identify or watermark the signal or image produced. In an alternative embodiment, the SAR signal is isolated so that it can be used. For example, the element or feature may be used to enrich an opto-acoustic image. In an embodiment, the element or feature or wavefront is used to produce an ultrasound image, which can be separately displayed or co-registered with a DAR image. In an embodiment, simulation, analytical calculation or experimentation and measurement is performed to describe acoustic wavefront(s) or waveform(s) produced by existing elements or features of the probe (or other component of the system). The processes and systems described above can then be modified to account for the "patterning" or "coding" of the existing system.

At least some of the resulting scatter may reach acoustic receivers, where it can be received and later processed as discussed above. In an embodiment, interfering codes are decoded by separating the mutually orthogonal code sequences and determining their relative intensities and acoustic propagations. In an embodiment, interfering codes can be removed from images and data using the technique of interframe persistent artifact removal. An example of interframe (or inter-frame) persistent artifact removal is described in WO2013056089. In an embodiment, the code can be detected, and a function of its intensity across the sequence of the code can be analyzed to provide information about the source intensity related to the illumination reaching the surface of the probe. In an embodiment, interframe persistent artifact removal may be applied after determining the intensities of the code, and then adaptively computing a static artifact removal frame. In an embodiment, the pattern may represent a chirp, a line-width modulated chirp (represented by a pattern of lines of different width), a grating, a tone, a linewidth modulated tone (represented by a pattern of lines of different width), or other such linewidth modulated pattern, including a sinc function or a wavelet. Dithering may be used on a pattern to permit a gradualized wavefront intensity. In an embodiment, the pattern may be dots or pattern elements (e.g., shapes) arranged on a grid or lattice. In an embodiment, the pattern on one side of the receiver array may differ or be offset from the pattern on the opposite side of the receiver array so that the ASW or other signals reaching the array can be differentiated. In an embodiment, features may be arranged on a triangular lattice, where lattice points on one side of the array are offset from mirroring lattice points on the other side of the array so that the side of the arriving ASW signal for a feature can be differentiated. In an embodiment, codes may be used to probe the properties of the epidermal layer or skin (thickness, roughness, optical or mechanical properties), or of the coupling medium.

A. Principle of creating features

In an embodiment, the probe or other component of the system is coded by modifying its geometry. For example, the shape, edges, flatness, convexity, surface, texture, width, height, length, depth, or orientation of an element or feature can be changed. In another embodiment, the probe or other component of the system is coded by modifying the color, reflectivity, transmissiveness, or absorption of electromagnetic energy of an element or feature. For example, in the case of light energy, a darker color can be selected that will absorb more light energy or the color can be matched to one or more wavelengths produced by the light source. The speed of sound, thermal expansion, and/or specific heat capacity of materials of optically absorbing elements or features on the probe or system component can also be manipulated to produce a pattern. These mechanical properties contribute to the opto-acoustic efficiency parameter, which is also known as the Gruneisen parameter. Such mechanical properties can affect the strength of a generated wavefront. In an embodiment, geometry can be used in conjunction with optical properties and/or mechanical properties of the element or feature. For example, colored bands could be added to a probe's face, which can shift the produced SAR signal in a wavelength dependent manner. In another embodiment, optical properties can applied in combination with mechanical properties. Other coding or changes to the probe will be apparent to one of skill in the art, and can be used in connection with the novel coded probe and the methods of component separation associated therewith without departing from the scope of the subject matter of the inventions disclosed herein.

Features that Block Light Exiting the Probe

In an embodiment, features, such as the features described above, are positioned at the light exit port or elsewhere in the light path. Such features can block or otherwise effect the light as it passes through the light exit port or other portion of the light path. Optically absorbing features directly in the path of the light exiting the exit port can have a different effect than similar optically absorbing features not in the light's direct path. In an embodiment, features in the light path absorb light or redirect or alter light without substantially absorbing it. In an embodiment, such features produce acoustic wavefronts. In the case of PASW, coded features can arrive at the acoustic receivers at the probe speed of sound, but may arrive at a different time through the coupling medium, or through the volume surface, which may have a variable speed of sound based on mechanical properties of the volume (e.g. a patient's skin), or operator applied pressure may alter the path length. Features directly in the light path can assist in removing interfering artifacts from light bars as light arrives at the volume. In another embodiment, a surface wave can be produced at a site located on the exit port that reduces the light delivered to a particular region of the volume. Other features blocking or otherwise affecting the light prior to the time it enters the volume will be apparent to one of skill in the art, and may be used in connection with the novel coded probe and component separation methods without departing from the scope of the inventions disclosed herein.

The present devices and methods are described above with reference to block diagrams and operational illustrations. It is understood that each block of the block diagrams or operational illustrations, and combinations of blocks in the block diagrams or operational illustrations, may be implemented by means of analog or digital hardware and computer program instructions. These computer program instructions may be provided to a processor of a general purpose computer, special purpose computer, ASIC, FPGA or other programmable data processing apparatus, such that the instructions, which execute via the processor of the computer or other programmable data processing apparatus, implements the functions/acts specified in the block diagrams or operational block or blocks. In some alternate implementations, the functions/acts noted in the blocks may occur out of the order noted in the operational illustrations. For example, two blocks shown in succession may in fact be executed substantially concurrently or the blocks may sometimes be executed in the reverse order, depending upon the functionality/acts involved.

As used in this description, "a" or "an" means "at least one" or "one or more" unless otherwise indicated. In addition, the singular forms "a", "an", and "the" include plural referents unless the content clearly dictates otherwise. Thus, for example, reference to a composition containing "a compound" includes a mixture of two or more compounds.

As used in this specification, the term "or" is generally employed in its sense including "and/or" unless the content clearly dictates otherwise.

The recitation herein of numerical ranges by endpoints includes all numbers subsumed within that range (e.g. 1 to 5 includes 1, 1.5, 2, 2.75, 3, 3.80, 4, and 5.

Unless otherwise indicated, all numbers expressing quantities of ingredients, measurement of properties and so forth used in the specification and claims are to be understood as being modified in all instances by the term "about," unless the context clearly dictates otherwise. Accordingly, unless indicated to the contrary, the numerical parameters set forth in the foregoing specification and attached claims are approximations that can vary depending upon the desired properties sought to be obtained by those skilled in the art utilizing the teachings of the present invention. At the very least, each numerical parameter should at least be construed in light of the number of reported significant digits and by applying ordinary rounding techniques. Any numerical value, however, inherently contains certain errors necessarily resulting from the standard deviations found in their respective testing measurements.

Those skilled in the art will recognize that the methods and devices of the present disclosure may be implemented in many manners and as such are not to be limited by the foregoing exemplary embodiments and examples. In other words, functional elements being performed by single or multiple components, in various combinations of hardware and software or firmware, and individual functions, may be distributed among software applications at either the client level or server level or both. In this regard, any number of the features of the different embodiments described herein may be combined into single or multiple embodiments, and alternate embodiments having fewer than, or more than, all of the features described herein are possible. Functionality may also be, in whole or in part, distributed among multiple components, in manners now known or to become known. Thus, myriad software/hardware/firmware combinations are possible in achieving the functions, features, interfaces and preferences described herein. Moreover, the scope of the present disclosure covers conventionally known manners for carrying out the described features and functions and interfaces, as well as those variations and modifications that may be made to the hardware or software or firmware components described herein as would be understood by those skilled in the art now and hereafter.

Furthermore, the embodiments of methods presented and described as flowcharts in this disclosure are provided by way of example in order to provide a more complete understanding of the technology. The disclosed methods are not limited to the operations and logical flow presented herein. Alternative embodiments are contemplated in which the order of the various operations is altered and in which sub-operations described as being part of a larger operation are performed independently.

It should be understood that the invention is not intended to be unduly limited by the specific embodiments and examples set forth herein, and that such embodiments and examples are presented merely to illustrate the invention, with the scope of the invention intended to be limited only by the claims attached hereto.

**Claims**

1. An opto-acoustic probe comprising:

   an acoustically transmissive optical distribution element (1360) comprising:

   a distal surface (1312) configured to couple to a volume of a biological tissue to deliver optical energy to the volume and to exchange acoustic energy with the volume; and,
   a proximal surface (1314) proximate to an acoustic receiver (1310),

   the acoustically transmissive optical distribution element configured to permit acoustic energy originating within the volume based on the optical energy delivered, to pass through the acoustically transmissive optical distribution element to be detected by the acoustic receiver;

wherein the acoustically transmissive optical distribution element is configured to receive the optical energy at one or more optical energy inputs from an optical energy path of the probe, and the acoustically transmissive optical distribution element is configured to distribute the optical energy received at the one or more optical energy inputs from the optical energy path to the distal surface, so that the optical energy distributed exits the distal surface of the acoustically transmissive optical distribution element

wherein the optical distribution element is configured to absorb at least a portion of the optical energy to produce a secondary acoustic return that interferes with a direct acoustic return component originating from the volume, wherein the opto-acoustic probe is operatively connected to an opto-acoustic system comprising a processing unit adapted to separate the secondary acoustic return from the direct acoustic return component using a component separation, and the system comprises a display to display an image based on the separated direct acoustic return component; and

wherein the optical distribution element comprises an optically diffusing fiber.

2. The opto-acoustic probe of claim 1, wherein the acoustically transmissive optical distribution element is an optically distributive acoustic lens, which acoustically transmissive optical distribution element comprises an optical energy input and the acoustically transmissive optical distribution element is configured to distribute optical energy, so that the optical energy exits the distal surface of the acoustically transmissive optical distribution element .

3. The opto-acoustic probe of claim 2, wherein the proximal surface of the acoustically transmissive optical distribution element is coated with an optically reflective coating that is acoustically transmissive.

4. The opto-acoustic probe of any of claims 1 to 3, wherein the distal surface of the acoustically transmissive optical distribution element is coated with an optically reflective coating that is acoustically transmissive.

5. The opto-acoustic probe of any preceding claim, wherein the optical distribution element is further configured to permit acoustic energy originating within the volume based on the optical energy delivered to pass through the optical distribution element to be detected by the acoustic receiver.

6. The opto-acoustic probe of any of claims 1 to 5, comprising a plastisol or gelatine.

7. The opto-acoustic probe of claim 6, comprising a plastisol mixed with a scattering agent such as titanium dioxide.

8. The opto-acoustic probe of any of claims 1 to 7, wherein the distal surface is coplanar with an exterior surface of the probe.

9. The opto-acoustic probe of any of claims 1 to 8, further configured to diffuse the optical energy received at the one or more optical energy inputs from the optical energy path to the distal surface.

10. The opto-acoustic probed of any preceding claim wherein the acoustically transmissive optical distribution element comprises a combined optical port and acoustic port, and the distal surface of the acoustically transmissive optical distribution element is coplanar with an exterior surface of the probe; and wherein the combined optical and acoustic port further comprises a protective layer.

**Patentansprüche**

1. Eine optoakustische Sonde, die Folgendes umfasst:
   ein schalldurchlässiges Lichtverteilungselement (1360), das Folgendes umfasst:

   eine distale Oberfläche (1312), die dazu ausgelegt ist, mit einem Volumen eines biologischen Gewebes gekoppelt zu werden, um dem Volumen Lichtenergie zuzuführen und Schallenergie mit dem Volumen auszutauschen; und
   eine proximale Oberfläche (1314) in der Nähe eines akustischen Empfängers (1310), wobei das schalldurchlässige Lichtverteilungselement dazu ausgelegt ist, der basierend auf der zugeführten Lichtenergie innerhalb des Volumens entstehenden Schallenergie zu erlauben, durch das schalldurchlässige Lichtverteilungselement hindurchzutreten, um von dem akustischen Empfänger detektiert zu werden;
   wobei das schalldurchlässige Lichtverteilungselement dazu ausgelegt ist, die Lichtenergie an einem oder mehreren Lichtenergieeingängen von einem Lichtenergieweg der Sonde zu empfangen, und wobei das schalldurchlässige Lichtverteilungselement dazu ausgelegt ist, die an dem einen oder den mehreren Lichtenergieeingängen von dem Lichtenergieweg empfangene Lichtenergie zu der distalen Oberfläche zu verteilen, sodass die verteilte Lichtenergie aus der distalen Oberfläche des schalldurchlässigen Lichtverteilungselements austritt; wobei das Lichtverteilungselement dazu ausgelegt ist, mindestens einen Anteil der Lichtenergie zu absorbieren, um ein sekundäres akustisches Rücksignal zu produzieren, das mit einer direkten akustischen Rücksignalkomponente, die aus dem Volumen heraus entsteht, interferiert, wobei die optoakustische Sonde betriebsfähig mit einem optoakustischen System verbunden ist, das eine Verarbeitungseinheit umfasst, die dazu angepasst ist, das sekundäre akustische Rücksignal von der direkten akustischen Rücksignalkomponente unter Verwendung einer Komponententrennung zu trennen, und wobei das System eine Anzeige umfasst, um ein Bild, das auf der getrennten direkten akustischen Rücksignalkomponente basiert, anzuzeigen; und wobei das Lichtverteilungselement eine optisch streuende Faser umfasst.

2. Optoakustische Sonde nach Anspruch 1, wobei das schalldurchlässige Lichtverteilungselement eine optisch verteilende akustische Linse ist, wobei das schalldurchlässige Lichtverteilungselement einen Lichtenergieeingang umfasst und das schalldurchlässige Lichtverteilungselement dazu ausgelegt ist, Lichtenergie zu verteilen, sodass die Lichtenergie aus der distalen Oberfläche des schalldurchlässigen Lichtverteilungselements austritt.

3. Optoakustische Sonde nach Anspruch 2, wobei die proximale Oberfläche des schalldurchlässigen Lichtverteilungselements mit einer optisch reflektierenden Beschichtung, die schalldurchlässig ist, beschichtet ist.

4. Optoakustische Sonde nach einem der Ansprüche 1 bis 3, wobei die distale Oberfläche des schalldurchlässigen Lichtverteilungselements mit einer optisch reflektierenden Beschichtung, die schalldurchlässig ist, beschichtet ist.

5. Optoakustische Sonde nach einem der vorhergehenden Ansprüche, wobei das Lichtverteilungselement ferner dazu ausgelegt ist, der basierend auf der zugeführten Lichtenergie innerhalb des Volumens entstehenden Schallenergie zu erlauben, durch das Lichtverteilungselement hindurchzutreten, um von dem akustischen Empfänger detektiert zu werden.

6. Optoakustische Sonde nach einem der Ansprüche 1 bis 5, die ein Plastisol oder eine Gelatine umfasst.

7. Optoakustische Sonde nach Anspruch 6, die ein Plastisol umfasst, das mit einem Streumittel wie etwa Titandioxid gemischt ist.

8. Optoakustische Sonde nach einem der Ansprüche 1 bis 7, wobei die distale Oberfläche koplanar mit einer äußeren Oberfläche der Sonde ist.

9. Optoakustische Sonde nach einem der Ansprüche 1 bis 8, die ferner dazu ausgelegt ist, die an dem einen oder den mehreren Lichtenergieeingängen empfangene Lichtenergie von dem Lichtenergieweg zu der distalen Oberfläche zu streuen.

10. Optoakustische Sonde nach einem der vorhergehenden Ansprüche, wobei das schalldurchlässige Lichtverteilungselement einen kombinierten optischen Anschluss und akustischen Anschluss umfasst und die distale Oberfläche des schalldurchlässigen Lichtverteilungselements koplanar mit einer äußeren Oberfläche der Sonde ist; und wobei der kombinierte optische und akustische Anschluss ferner eine Schutzschicht umfasst.

## Revendications

1. Sonde opto-acoustique comprenant :
   un élément de distribution optique à transmission acoustique (1360) comprenant :

   une surface distale (1312) configurée pour un couplage à un volume d'un tissu biologique pour apporter de l'énergie optique au volume et pour échanger de l'énergie acoustique avec le volume ; et
   une surface proximale (1314) située à proximité d'un récepteur acoustique (1310), l'élément de distribution optique à transmission acoustique étant configuré pour permettre à de l'énergie acoustique provenant de l'intérieur du volume, en fonction de l'énergie optique apportée, de passer à travers l'élément de distribution optique à transmission acoustique pour être détectée par le récepteur acoustique ;

dans laquelle l'élément de distribution optique à transmission acoustique est configuré pour recevoir l'énergie optique au niveau d'une ou plusieurs entrées d'énergie optique à partir d'un chemin d'énergie optique de la sonde, et l'élément de distribution optique à transmission acoustique est configuré pour distribuer l'énergie optique reçue au niveau des une ou plusieurs entrées d'énergie optique du chemin d'énergie optique à la surface distale, de telle sorte que l'énergie optique distribuée sorte de la surface distale de l'élément de distribution optique à transmission acoustique ;

dans laquelle l'élément de distribution optique est configuré pour absorber au moins une partie de l'énergie optique pour produire un retour acoustique secondaire qui interfère avec un composant de retour acoustique direct provenant du volume, la sonde opto-acoustique étant fonctionnellement connectée à un système opto-acoustique comprenant une unité de traitement conçue pour séparer le retour acoustique secondaire du composant de retour acoustique direct par séparation des composants, et le système comprend un affichage permettant d'afficher une image basée sur le composant de retour acoustique direct séparé ; et

dans laquelle l'élément de distribution optique comprend une fibre de diffusion optique.

2. Sonde opto-acoustique selon la revendication 1, dans laquelle l'élément de distribution optique à transmission acoustique est une lentille acoustique de distribution optique, lequel élément de distribution optique à transmission acoustique comprend une entrée d'énergie optique et l'élément de distribution optique à transmission acoustique est configuré pour distribuer de l'énergie optique, de telle sorte que l'énergie optique sorte de la surface distale de l'élément de distribution optique à transmission acoustique.

3. Sonde opto-acoustique selon la revendication 2, dans laquelle la surface proximale de l'élément de distribution optique à transmission acoustique est recouverte d'un revêtement optiquement réfléchissant qui est à transmission acoustique.

4. Sonde opto-acoustique selon l'une quelconque des revendications 1 à 3, dans laquelle la surface distale de l'élément de distribution optique à transmission acoustique est recouverte d'un revêtement optiquement réfléchissant qui est à transmission acoustique.

5. Sonde opto-acoustique selon l'une quelconque des revendications précédentes, dans laquelle l'élément de distribution optique est en outre configuré pour permettre à l'énergie acoustique provenant de l'intérieur du volume, en fonction de l'énergie optique apportée, de passer à travers l'élément de distribution optique pour être détectée par le récepteur acoustique.

6. Sonde opto-acoustique selon l'une quelconque des revendications 1 à 5, comprenant un plastisol ou de la gélatine.

7. Sonde opto-acoustique selon la revendication 6, comprenant un plastisol mélangé avec un agent de diffusion tel que le dioxyde de titane.

8. Sonde opto-acoustique selon l'une quelconque des revendications 1 à 7, dans laquelle la surface distale est coplanaire avec une surface extérieure de la sonde.

9. Sonde opto-acoustique selon l'une quelconque des revendications 1 à 8, configurée en outre pour diffuser l'énergie optique reçue au niveau des une ou plusieurs entrées d'énergie optique du chemin d'énergie optique à la surface distale.

10. Sonde opto-acoustique selon l'une quelconque des revendications précédentes, dans laquelle l'élément de distribution optique à transmission acoustique comprend un port optique et un port acoustique combinés, et la surface distale de l'élément de distribution optique à transmission acoustique est coplanaire avec une surface extérieure de la sonde ; et dans laquelle le port optique et acoustique combiné comprend en outre une couche protectrice.

FIG. 1

FIG. 2

FIG. 3

FIG. 4

FIG. 5

FIG. 6

FIG. 7

FIG. 8A

FIG. 8B

a) FIG. 9A

b) FIG. 9B

c) FIG. 9C

d) FIG. 9D

FIG. 10

DAR reconstruction — DAR target — interference from SAR target

component separated DAR

component separated DAR (thresholded)

PSF (DAR channel)

a)

b)

c)

d)

SAR reconstruction — interference from DAR target — SAR target

component separated SAR

component separated SAR (thresholded)

PSF (SAR channel)

e)

f)

g)

h)

FIG. 11

EP 4 018 918 B1

FIG. 12A

FIG. 12B

FIG. 12C

```
┌─────────────────────────────────────────────┐
│         Deliver electromagnetic energy        │
└─────────────────────────────────────────────┘
                       │
                       ▼
┌─────────────────────────────────────────────┐
│   Receive a multiple component acoustic signal │
│         at all active detector positions       │
└─────────────────────────────────────────────┘
                       │
                       ▼
┌─────────────────────────────────────────────┐
│      Construct a reference representation      │
│          for each component of signal          │
└─────────────────────────────────────────────┘
                       │
                       ▼
┌─────────────────────────────────────────────┐
│       Initialize a current representation      │
│          for each component of signal          │
└─────────────────────────────────────────────┘
                       │
                       ▼
┌─────────────────────────────────────────────┐
│  ┌───────────────────────────────────────┐   │
│  │  Apply point spread function to each current │
│  │  representation to create PSF representation │
│  │  └───────────────────────────────────┘   │
│  │                   │                       │
│  │                   ▼                       │
│  │  ┌───────────────────────────────────┐   │
│  │  │ Calculate residual error from reference │
│  │  │ representations and PSF representation │
│  │  └───────────────────────────────────┘   │
│  │                   │                       │
│  │                   ▼                       │
│  │  ┌───────────────────────────────────┐   │
│  │  │      Correct current representations    │
│  │  │       based on calculated residuals     │
│  │  └───────────────────────────────────┘   │
│  │                   │                       │
│  │                   ▼                       │
│  │  ┌───────────────────────────────────┐   │
│  │  │         Apply thresholding             │
│  └──┴───────────────────────────────────┘   │
└─────────────────────────────────────────────┘
                       │
                       ▼
┌─────────────────────────────────────────────┐
│        Output and/or store representations     │
└─────────────────────────────────────────────┘
```

# FIG. 13

Construct a reference representation
for each component of signal for each frame

Initialize a current representation
for each component of signal for each frame

Apply point spread function to each current
representation to create PSF representation

Calculate residual error from reference
representations and PSF representation

Correct current representations
based on calculated residuals

Apply thresholding

## FIG. 14A

Construct a reference representation
for each component of signal for each frame

Initialize a current representation
for each component of signal for each frame

Apply dictionary transformation to each current
represenation and/or each reference representation

Apply point spread function to each current
representation to create PSF representation

Apply inverse dictionary transformation to
each current represenation

Apply point spread function

Apply dictionary transformation to each
current represenation

Calculate residual error from reference
representations and PSF representation

Correct current representations
based on calculated residuals

Apply thresholding

## FIG. 14B

Construct a reference representation
for each component of signal for each frame

Initialize a current representation
for each component of signal for each frame

Apply independent subband dictionary transformation to
each current represenation and/or each reference
representation to create sub-band representations

Apply sub-band point spread function to each
current sub-band representation to create PSF
sub-band representation

Calculate residual error from sub-band reference
representations and PSF sub-band
representation

Correct current sub-band representations
based on calculated residuals

Apply thresholding

Apply inverse subband dictionary transformation
to independent sub-bands

Output overall result

## FIG. 14C

Construct a reference representation
for each component of signal for each frame

Initialize a current representation
for each component of signal for each frame

Apply dictionary transformation to each current
represenation and/or each reference representation

Apply point spread function to each current
representation to create PSF representation

Calculate residual error from reference
representations and PSF representation

Correct current representations
based on calculated residuals

Apply dictionary transformation to each
current represenation

Apply thresholding

Apply inverse dictionary transformation to each
current represenation

## FIG. 14D

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61945650
- US 2013197345 A1
- US 20130281819 A
- WO 2013056089 A
- US 20140206978 A

**Non-patent literature cited in the description**

- **GUERQUIN-KERN**. A Fast Wavelet-Based Reconstruction Method for Magnetic Resonance Imaging. *IEEE Transactions on Medical Imaging*, 2011, vol. 30 (9), 1649
- **BOBIN et al.** Morphological Diversity and Sparsity for Multichannel Data Restoration. *Journal of Mathematical Imaging and Vision*, February 2009, vol. 33 (2), 149-168